(19) Europäisches Patentamt / European Patent Office / Office européen des brevets

(11) **EP 3 764 102 A1**

(12) **EUROPEAN PATENT APPLICATION**
published in accordance with Art. 153(4) EPC

(43) Date of publication:
**13.01.2021 Bulletin 2021/02**

(21) Application number: **19764930.4**

(22) Date of filing: **08.03.2019**

(51) Int Cl.:
*G01N 33/68* (2006.01)   *G01N 27/62* (2021.01)

(86) International application number:
**PCT/JP2019/009370**

(87) International publication number:
**WO 2019/172427 (12.09.2019 Gazette 2019/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.03.2018   JP 2018043641**
**24.07.2018   JP 2018138785**

(71) Applicants:
• **National University Corporation Tokyo Medical and Dental University**
**Tokyo 113-8510 (JP)**
• **Denka Company Limited**
**Tokyo 103-8338 (JP)**

(72) Inventor: **OKAZAWA Hitoshi**
**Tokyo 113-8510 (JP)**

(74) Representative: **Mewburn Ellis LLP**
**Aurora Building**
**Counterslip**
**Bristol BS1 6BX (GB)**

(54) **DETECTION OF ALZHEIMER'S DISEASE (AD), FRONTOTEMPORAL LOBAR DEGENERATION (FTLD), AMYOTROPHIC LATERAL SCHLEROSIS (ALS), PARKINSON'S DISEASE (PD), AND DEMENTIA WITH LEWY BODIES (DLB) INDICATED BY PHOSPHORYLATION OF MARCKS**

(57)   Provided is a method for detecting a neurodegenerative disease selected from the group consisting of human Alzheimer's disease (AD), frontotemporal lobar degeneration (FTLD), and amyotrophic lateral sclerosis (ALS) with high sensitivity and high specificity. A method for detecting a neurodegenerative disease selected from the group consisting of human Alzheimer's disease (AD), frontotemporal lobar degeneration (FTLD), and amyotrophic lateral sclerosis (ALS), comprising the steps of: (i) measuring a MARCKS protein phosphorylated at position 46 and a non-phosphorylated MARCKS protein in a test specimen collected from a subject; (ii) calculating a DO value expressed by the following formula from the measured values obtained in (i):

[Formula 1]

$$DO = \sqrt{(pSer46\text{-}MARCKS)^2 + (non\text{-}phosphorylated\text{-}MARCKS)^2}$$

where "pSer46-MARCKS" represents the amount of a MARCKS protein phosphorylated at position 46; and "non-phosphorylated-MARCKS" represents the amount of a non-phosphorylated MARCKS protein; and (iii) detecting a neurodegenerative disease based on the DO value as an indicator.

EP 3 764 102 A1

## Description

Technical Field

The present invention relates to a method for detecting Alzheimer's disease (AD), frontotemporal lobar degeneration (FTLD), amyotrophic lateral sclerosis (ALS), Parkinson's disease (PD), and dementia with Lewy bodies (DLB).

Background Art

Preclinical pathology of neurodegenerative diseases has attracted a lot of attention and development of a biomarker quantitatively determining early-stage pathological conditions of these diseases has been required.

Alzheimer's disease (AD) is the most common neurodegenerative disease and a well-known cause of dementia.

One of the most supportive models about the pathogenesis of AD is an amyloid hypothesis on the premise of cytotoxicity of amyloid fibrils, which is produced by extracellular accumulation of amyloid β peptide (Aβ).

Based on the hypothesis, Aβ has come up as a main target of AD treatment. Therapeutic strategies including a therapy with an antibody such as bapineuzumab and solanezumab reduce Aβ aggregation in the brains of human AD patients in clinical trials carried out after onset of dementia; however, recovery from dementia as clinical signs is insufficient up to present. As a result, researchers' interest turns towards molecular events that occur in the early and presymptomatic stage of AD for which an analogous or new therapy may be more effective.

The present inventors conducted comprehensive analyses of phosphorylated proteins in the brain tissues taken from mouse AD models and human AD patients. They identified 17 core proteins abnormally phosphorylated in a plurality of AD mouse models. Interestingly, these changes occurred before onset of AD; besides, in some of the core proteins, abnormal phosphorylation was detected even before Aβ aggregation was immunohistologically detected in model mouse brains, suggesting that the abnormal phosphorylation plays an important role in elucidating pathology in the early stage of AD (see Non Patent Literature 1).

Particularly in quantitative mass spectrometry, MARCKS (myristoylated alanine-rich protein kinase C substrate) phosphorylated at Ser46 started increasing a month before Aβ aggregation was histologically detected in AD model mice and remained even in the dead brain of a human AD patient (see Non Patent Literatures 1 and 2). MARCKS is a submembrane protein, which fixes an actin cell skeletal network and a substrate mostly present in protein kinase C (PKC). In an immunohistochemical analysis using anti-pSer46-MARCKS, dystrophic neurite was detected in AD model mice, far before symptoms appeared, and dystrophic neurites surrounding senile spots were detected in human autopsy AD brain (see Non Patent Literature 2). Furthermore, it was elucidated that phosphorylation of MARCKS at Ser46 destabilizes dendrite spines and causes neurite degeneration (see Non Patent Literature 2). These results proposed a hypothesis that pSer46-MARCKS can be used as a biomarker substitute for neurite degeneration, which can be used until the terminal stage of AD and presumably from super-early or preclinical stage in other neurodegenerative diseases.

Citation List

Non Patent Literature

Non Patent Literature 1: Tagawa, K. et al., Hum. Mol. Genet. 24, 540-58 (2015).
Non Patent Literature 2: Fujita, K. et al., Sci. Rep. 6, 31895 (2016).

Summary of Invention

Technical Problem

An object of the present invention is to provide a method for detecting a neurodegenerative disease selected from the group consisting of human Alzheimer's disease (AD), frontotemporal lobar degeneration (FTLD), amyotrophic lateral sclerosis (ALS), Parkinson's disease (PD), and dementia with Lewy bodies (DLB) or Lewy body disease with high sensitivity and high specificity.

Solution to Problem

As described above, the present inventors previously found that phosphorylated MARCKS increases even before amyloid β (Aβ) aggregates are histologically formed in Alzheimer's disease (AD) mouse models, and that such a change is maintained in the dead human AD brain. Particularly MARCKS phosphorylated at Ser46 (pSer46-MARCKS) was detected in a human-patient mouse model and dystrophic neurites surrounding Aβ aggregate of a senile spot.

Based on these findings, the present inventors measured the amount of pSer46-MARCKS in the cerebrospinal fluids (CSFs) of human Alzheimer's disease (AD), frontotemporal lobar degeneration (FTLD), and amyotrophic lateral sclerosis (ALS) patients. Unexpectedly, the most remarkable change was found in frontotemporal lobar degeneration (FTLD) and neurite degeneration was confirmed by an immunohistochemical analysis of pSer46-MARCKS. Specificity of the cerebrospinal fluid (CSF) on pSer46-MARCKS is 1.0. From this, it was found that neurodegeneration can be distinguished from a normal case.

It was further found that the same change in MARCKS occurs in Parkinson's disease and dementia with Lewy bodies not only in mouse models but also in human patients.

At the time point when a humanized α-Syn-BAC-Tg/GBA-hetero-KO mouse did not show a symptom and before α-synuclein aggregate was formed, the level of pSer46-MARCKS started increasing and the increased level was maintained during the aging process. This phenomenon matched with post-mortem pattern of a human brain.

The result strongly suggests that a common mechanism underlies between AD and PD/DLB lesions, i.e., pre-aggregation neurite degeneration.

Furthermore, the same change was observed in both mouse models and human patients having Parkinson's disease (PD) and dementia with Lewy bodies (DLB).

Moreover, the present inventors quantified pSer46-MARCKS and non-phosphorylated MARCKS in the cerebrospinal fluids (CSFs) taken from human patients having AD and patients having other neurodegenerative diseases, by mass spectrometry. The sensitivity and specificity of pSer46-MARCKS itself are both sufficiently high. In an attempt to further enhance the value of pSer46-MARCKS as a diagnostic parameter, the present inventors developed a new parameter by integration of pSer46-MARCKS and non-phosphorylated MARCKS. The new parameter made it possible to distinguish a plurality of neurodegenerative diseases from healthy controls with high sensitivity and high specificity. Pathological analysis was further conducted and clarified that pSer46-MARCKS reflects neurite degeneration of all neurodegenerative diseases. Based on these results, a new parameter, which is called "DO (distance from origin)" and serves as a highly sensitive and definitive biomarker for all neurodegenerative diseases, was developed by integration of pSer46-MARCKS and non-phosphorylated MARCKS.

More specifically, the present invention is as follows.

[1] A method for detecting a neurodegenerative disease selected from the group consisting of human Alzheimer's disease (AD), frontotemporal lobar degeneration (FTLD), and amyotrophic lateral sclerosis (ALS), comprising the steps of:

(i) measuring a MARCKS protein phosphorylated at position 46 and a non-phosphorylated MARCKS protein in a test specimen collected from a subject;
(ii) calculating a DO value expressed by the following formula from the measured values obtained in (i):

[Formula 1]

$$DO = \sqrt{(pSer46\text{-}MARCKS)^2 + (non\text{-}phosphorylated\text{-}MARCKS)^2}$$

where "pSer46-MARCKS" represents the amount of a MARCKS protein phosphorylated at position 46; and "non-phosphorylated-MARCKS" represents the amount of a non-phosphorylated MARCKS protein; and
(iii) detecting a neurodegenerative disease based on the DO value as an indicator.

[2] The method according to [1], wherein the test specimen is cerebrospinal fluid.
[3] The method according to [1] or [2], wherein "pSer46-MARCKS" and "non-phosphorylated-MARCKS" are measured by mass spectrometry.
[4] The method according to any one of [1] to [3], wherein "pSer46-MARCKS" and "non-phosphorylated-MARCKS" in the formula are normalized values by the total amount of proteins in the test specimen.
[5] The method according to any one of [1] to [4], wherein, in step (iii), the DO value of the subject calculated is compared to a cutoff value previously specified and if the DO value is higher than the cutoff value, it is determined that a neurodegenerative disease is detected.
[6] The method according to [5], wherein the cutoff value of the DO value is 50 to 55.
[7] A method for detecting a neurodegenerative disease selected from the group consisting of human frontotemporal lobar degeneration (FTLD), amyotrophic lateral sclerosis (ALS), Parkinson's disease (PD), and dementia with Lewy bodies (DLB), comprising the steps of:

(i) measuring a MARCKS protein phosphorylated at position 46 in a test specimen collected from a subject;
(ii) comparing the amount of the MARCKS protein phosphorylated at position 46 in the test specimen collected from the subject to the amount of the MARCKS protein phosphorylated at position 46 in a test specimen collected from a healthy person; and
(iii) determining that a neurodegenerative disease is detected in the subject if the amount of the MARCKS protein phosphorylated at position 46 in the test specimen collected from the subject is larger than the amount of the MARCKS protein phosphorylated at position 46 in the test specimen collected from the healthy person.

[8] The method according to [7], wherein the test specimen is cerebrospinal fluid.
[9] The method according to [7] or [8], wherein the MARCKS protein phosphorylated at position 46 is measured by mass spectrometry.
[10] The method according to any one of [7] to [9], wherein the amount of the MARCKS protein phosphorylated at position 46 is a value obtained by normalizing the measured value of the MARCKS protein phosphorylated at position 46 by the total amount of proteins in the test specimen or the total amount of MARCKS proteins in the test specimen.
[11] A method for detecting a neurodegenerative disease selected from the group consisting of Parkinson's disease (PD) and dementia with Lewy bodies (DLB), comprising the steps of:

(i) measuring a MARCKS protein phosphorylated at position 46 in test specimens collected from the occipital lobe and temporal lobe of a subject;
(ii) comparing the amounts of the MARCKS protein phosphorylated at position 46 in the test specimens collected from the occipital lobe and temporal lobe of the subject to the amounts of the MARCKS protein phosphorylated at position 46 in test specimens collected from the occipital lobe and temporal lobe of a healthy person; and
(iii) determining that a neurodegenerative disease is detected in the subject if the amount of the MARCKS protein phosphorylated at position 46 in the test specimen collected from the temporal lobe of the subject is larger than the amount of the MARCKS protein phosphorylated at position 46 in the test specimen collected from the temporal lobe of the healthy person.

[12] The method according to [11], wherein the test specimen is cerebrospinal fluid.
[13] The method according to [11] or [12], wherein the MARCKS protein phosphorylated at position 46 is measured by mass spectrometry.
[14] The method according to any one of [11] to [13], wherein the amount of the MARCKS protein phosphorylated at position 46 is a value obtained by normalizing the measured value of the MARCKS protein phosphorylated at position 46 by the total amount of proteins in the test specimen or the total amount of MARCKS proteins in the test specimen.
[15] The method according to [11], wherein the MARCKS protein phosphorylated at position 46 is measured by positron emission tomography (PET) using a PET tracer for imaging the MARCKS protein phosphorylated at position 46.
[16] The method according to [15], wherein the PET tracer for imaging the MARCKS protein phosphorylated at position 46 is an antibody PET tracer prepared by labeling an antibody to the MARCKS protein phosphorylated at position 46 with a positron nuclide.

The present specification includes the disclosures of JP Patent Application Nos. 2018-043641 and 2018-138785, based on which the priority of the present application is claimed.

Advantageous Effects of Invention

The method of the present invention makes it possible to detect a neurodegenerative disease selected from the group consisting of human Alzheimer's disease (AD), frontotemporal lobar degeneration (FTLD), amyotrophic lateral sclerosis (ALS), Parkinson's disease (PD), and dementia with Lewy bodies (DLB) with high sensitivity and high specificity.

Brief Description of Drawings

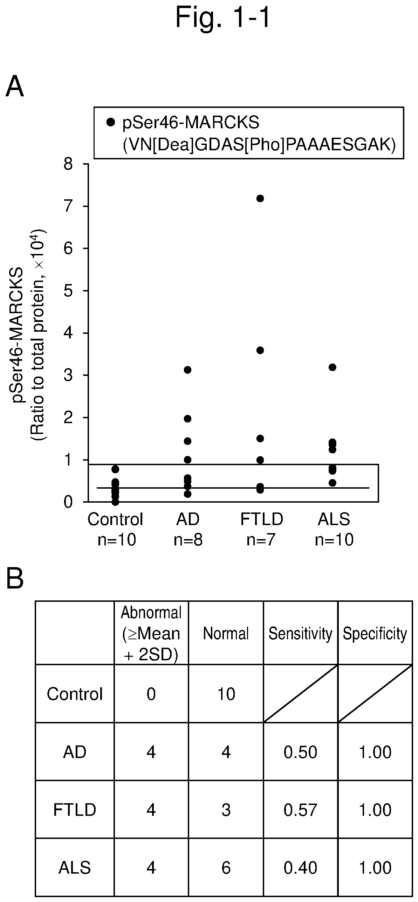

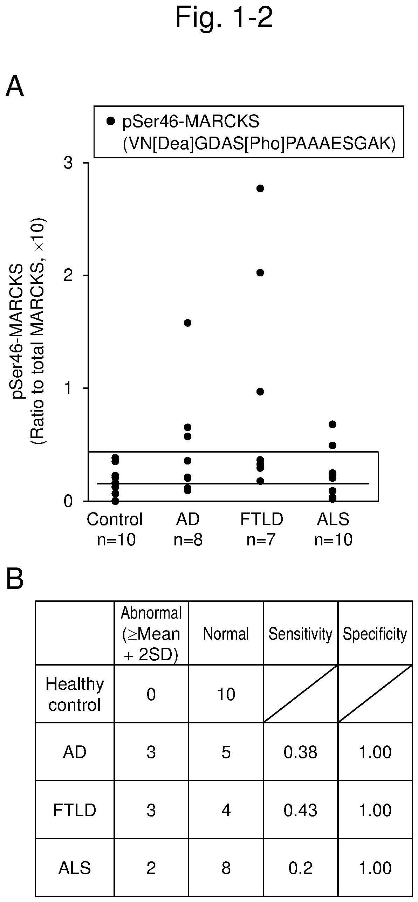

[Figure 1-1] The figures show the evaluation results of pSer46-MARCKS in the cerebrospinal fluid (CSF) as a biomarker for diagnosis. Figure 1-1A is shown by normalized values of the amounts of pSer46-MARCKS in individual neurodegenerative disease groups by the total amount of proteins; and Figure 1-1B shows sensitivity and specificity.

[Figure 1-2] The figures show the evaluation results of pSer46-MARCKS in the cerebrospinal fluid (CSF) as a biomarker for diagnosis. Figure 1-2A is shown by normalized values of the amounts of pSer46-MARCKS in individual neurodegenerative disease groups by the total amount of MARCKS proteins; and Figure 1-2B shows sensitivity and specificity.

[Figure 2-1] The figures show the evaluation results of non-phosphorylated MARCKS in the cerebrospinal fluid (CSF) as a biomarker for diagnosis. Figure 2-1A is shown by normalized values of the amounts of non-phosphorylated MARCKS in individual neurodegenerative disease groups by the total amount of proteins; and Figure 2-1B shows sensitivity and specificity.

[Figure 2-2] The figures show the evaluation results of non-phosphorylated MARCKS in the cerebrospinal fluid (CSF) as a biomarker for diagnosis. Figure 2-2A is shown by normalized values of the amounts of non-phosphorylated MARCKS in individual neurodegenerative disease groups by the total amount of MARCKS proteins; and Figure 2-2B shows sensitivity and specificity.

[Figure 3-1] The figures show the amounts of pSer46-MARCKS in AD, FTLD and ALS patients. Figure 3-1A shows the normalized amounts by the total protein; and Figure 3-1B shows the normalized values by the total amount of MARCKS.

[Figure 3-2] The figure shows the amount of non-phosphorylated MARCKS in AD, FTLD and ALS patients. Figure 3-2A shows the normalized amounts by the total protein; and Figure 3-2B shows the normalized values by the total amount of MARCKS.

[Figure 4-1] The figures show the relationship between pSer46-MARCKS and non-phosphorylated MARCKS in AD, FTLD and ALS patients when the values are normalized by the total amount of proteins; and the figures are shown for each disease group (A: control, B: AD, C: FTLD, D: ALS).

[Figure 4-2] The figure collectively shows the relationships of A to D shown in Figure 4-1.

[Figure 4-3] The figures show the relationship between pSer46-MARCKS and non-phosphorylated MARCKS in AD, FTLD and ALS patients when the values are normalized by the total amount of MARCKS; and the figures show separately per disease group (A: control, B: AD, C: FTLD, D: ALS).

[Figure 4-4] The figure collectively shows the relationships of A to D shown in Figure 4-3.

[Figure 5-1] The figure shows the relationship between HMGB1 and pSer46-MARCKS in the cerebrospinal fluid (CSF) (pSer46-MARCKS relative to the total amount of proteins).

[Figure 5-2] The figure shows the relationship between HMGB 1 and pSer46-MARCKS in the cerebrospinal fluid (CSF) (pSer46-MARCKS relative to the total amount of MARCKS).

[Figure 6-1] The figure shows stained image of pTDP-43 of human FTLD.

[Figure 6-2] The figure shows the results of immunohistochemical staining of human FTLD brain with an anti-pSer46-MARCKS antibody.

[Figure 6-3] The figure shows the results of co-staining of the human FTLD occipital lobe (occipital cortex) with MAP2 and pSer46-MARCKS.

[Figure 7] The figure shows disappearance of neurons and sponge-like degeneration of the frontal lobe (frontal cortex) and occipital lobe (occipital cortex) in human FTLD.

[Figure 8-1] The figure shows the results of the ideal "upper limit of normal DO" searched by numerical simulation.

[Figure 8-2] The figure shows DO values of individual diseases.

[Figure 8-3] The figure shows the number of cases determined as being abnormal in control, AD, FTLD and ALS groups, target scores, and values of sensitivity and specificity at the maximum optimization time in the DO range.

[Figure 9] The figures each show the correlation between DO and disease severity in view of clinical symptoms.

[Figure 10A] The figure shows results of comprehensive phosphoproteome analysis of human AD and DLB dead brain, more specifically, the comparative results of independent phosphorylation sites between AD and DLB.

[Figure 10B] The figure shows results of comprehensive phosphoproteome analysis of human AD and DLB dead brains, more specifically, comparison of changes in 4 different phosphorylation sites [Ser27/Ser27, Ser46/Ser46, Ser145/Ser138 and Thr150/Thr143 (human/mouse)] of MARCKS between human DLB patients and AD patients.

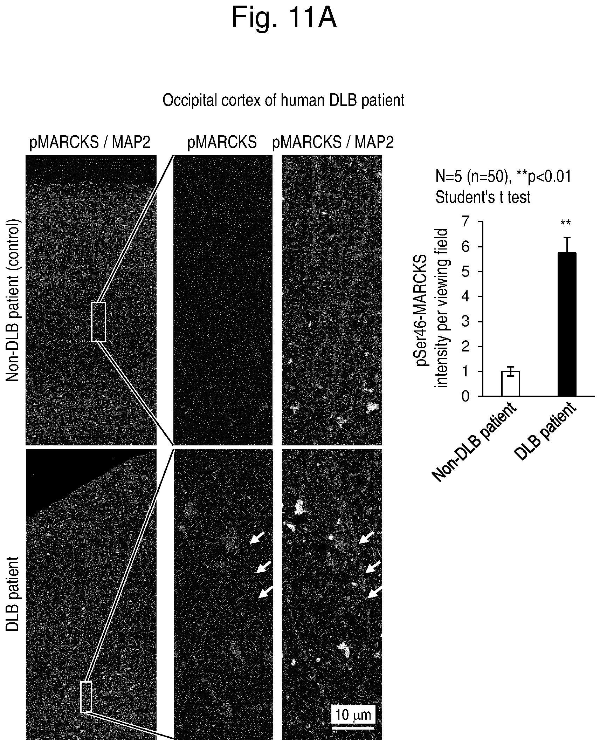

[Figure 11A] The figure shows immunohistochemical analysis of human DLB brain, more specifically, simultaneous staining results of pSer46-MARCKS and MAP2. Low magnification images of (panels of the leftmost row) and high magnification images (panels of two right rows) of temporal lobes taken from non-DLB control patients (non-DLB patient) (5 females) and human DLB patients (5 females). Images were obtained by Olympus FV1200 IX83 confocal microscopy. All bar charts show mean values and S.E.M. Signal intensities (average pixel intensity) in 10 viewing fields ($100 \times 100 \ \mu m$) randomly selected from the corresponding regions in individual patients were quantitatively analyzed. Statistical analysis was carried out using Student's t test. Reference symbol ** indicates p < 0.01.

[Figure 11B] The figure shows immunohistochemical analysis of human DLB brain. Staining of pSer129-$\alpha$-Syn clearly showed the presence of a plurality of cytoplasmic inclusion bodies (Lewy bodies) in the same patient group.

[Figure 11C] The figure shows immunohistochemical analysis of human DLB brain, more specifically, the results of simultaneous staining of ubiquitin and Ser129-α-Syn.

[Figure 11D] The figure shows immunohistochemical analysis of human DLB brain, more specifically, the results of co-staining of pSer46-MARCKS and pSer129-α-Syn.

[Figure 11E] The figure shows immunohistochemical analysis of human DLB brain, more specifically, the results of western blot analysis of the occipital lobe derived from a non-DLB control patient and a DLB patient having antibodies to pSer46-MARCKS, total MARCKS and β-actin. The graph shows quantitative results of pSer46-MARCKS from 5 patients and 5 non-neurological disease patients (control). The intensity of bands was normalized by that of β-actin. The statistical analysis was carried out using Student's t test. Reference symbol * indicates $p < 0.05$.

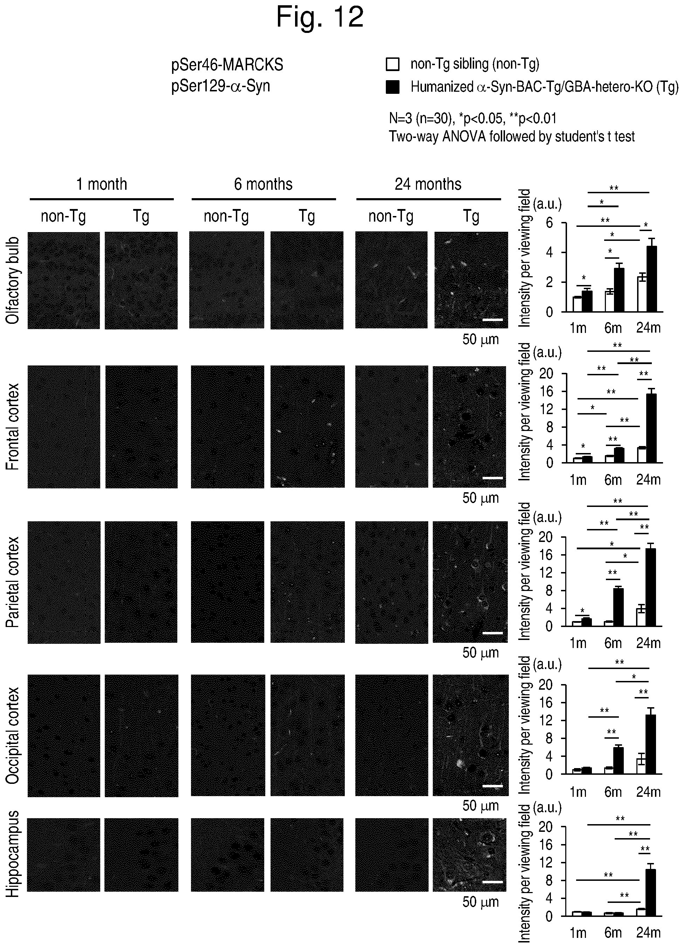

[Figure 12] The figure shows a time-dependent change of pSer46-MARCKS in human. α-Syn-BAC-Tg/GBA-hetero-KO mouse pSer46-MARCKS and pSer129-α-Syn were simultaneously stained in humanized normal α-Syn-BAC-Tg/glucocerebrosidase (GBA)-hetero-KO mice of 1, 6, and 24 months old (3 male mice for each time point). Images were obtained by Olympus FV1200 IX83 confocal microscopy. All bar charts show mean values and S.E.M. 3 mice per group were used and the signal intensity (average pixel intensity) was quantitatively analyzed in 10 viewing fields ($100 \times 100$ μm) for each randomly selected from the brain region thereof. The statistical analysis was carried out using two-way ANOVA followed by Student's t test. Reference symbol * indicates $p < 0.05$ and reference symbol ** indicates $p < 0.01$. An increase of phosphorylation was first found in the olfactory bulb and the frontal cortex, and subsequently in the temporal cortex and the occipital cortex. The signal intensity of pSer46-MARCKS rapidly increased in the temporal and occipital regions. The signal intensity of these regions was the strongest in a plurality of brain regions.

[Figure 13A] The figure shows the results of co-staining of pSer46-MARCKS and pSer129-α-syn in the olfactory bulb of a humanized α-Syn-BAC-Tg/GBA-hetero-KO mouse (1 month old).

[Figure 13B] The figure shows the results of co-staining of pSer46-MARCKS and pSer129-α-syn in the olfactory bulb of a humanized α-Syn-BAC-Tg/GBA-hetero-KO mouse (6 months old).

[Figure 13C] The figure shows the results of co-staining of pSer46-MARCKS and pSer129-α-syn in the olfactory bulb of a humanized α-Syn-BAC-Tg/GBA-hetero-KO mouse (24 months old).

[Figure 13D] The figure shows staining of ubiquitin in the olfactory bulb of a humanized α-Syn-BAC-Tg/GBA-hetero-KO mouse. Ubiquitin was co-stained as a dot-like structure or a cytoplasm aggregate in a cell subset (yellow arrow), and pSer129-α-Syn-positive/ubiquitin-negative dots or aggregates were also observed.

[Figure 14A] The figure shows immunohistochemical analysis of the parietal lobe of α-Syn-BAC-Tg/GBA-hetero-KO mouse. In the cases of pSer46-MARCKS and pSer129-α-Syn, the external and internal pyramid cell layers of a 24-month-old mouse were co-stained. In the case of pSer46-MARCKS, the dendrite of the apex and cell body were both stained; however in the case of pSer129-α-Syn, a cytoplasm aggregate was stained.

[Figure 14B] The figure shows immunohistochemical analysis of the parietal lobe of α-Syn-BAC-Tg/GBA-hetero-KO mouse, more specifically, staining of the parietal cortex tissue derived from humanized α-Syn-BAC-Tg/GBA-hetero-KO mouse. In 1-, 6-, and 24-month-old mice, pSer129-α-Syn and pSer46-MARCKS or ubiquitin were co-stained. The same co-staining pattern as in the olfactory bulb was observed in the parietal lobe.

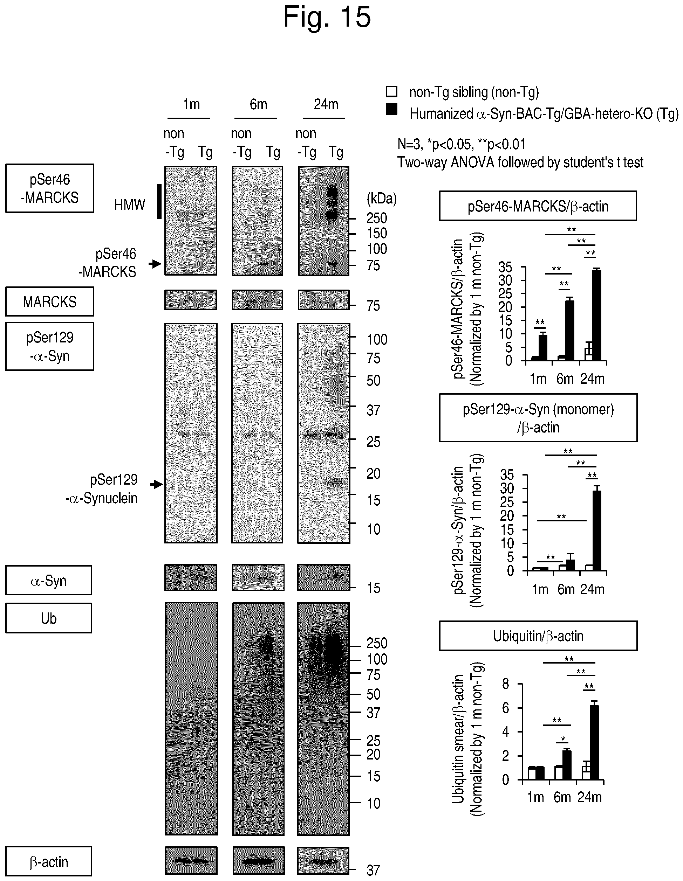

[Figure 15] The figure shows the results of western blot analysis of the olfactory bulb of α-Syn-BAC-Tg/GBA-hetero-KO mouse at a plurality of time points. The left panels (3 rows) show the results of western blot of the whole cortex by using antibodies to pSer46-MARCKS, pSer129-α-Syn and ubiquitin (3 male mice per each time point). The right graphs show the quantitative analysis results of three independent blots of pSer46-MARCKS, pSer129-α-Syn and ubiquitin. The intensity of bands was normalized by that of β-actin. The statistical analysis was carried out by two-way ANOVA followed by Student's t test; * $p < 0.05$, **$p < 0.01$.

[Figure 16] The figures show the interaction between MARKCS and α-syn. Figure 16A shows the results of blotting an immunoprecipitate, which was obtained from a brain sample of α-Syn-BAC-Tg/GBA-hetero-KO mouse with an anti-pSer46-MARCKS or anti-pSer129-α-Syn antibody, with an anti-pSer129-α-Syn or anti-pSer46-MARCKS antibody, at time points of 1, 6, and 24 months; and Figure 16B shows the results of a co-precipitate obtained in the same manner as above from the occipital cortex of a human DLB patient.

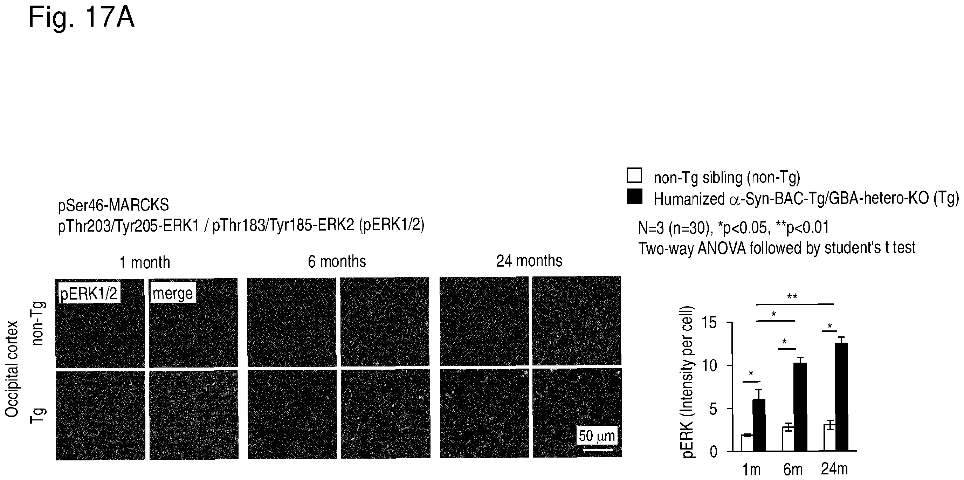

[Figure 17A] The figure shows activation of Erk1/2 in the cortical neurons of a mouse PD/DLB model and a human DLB patient. Identification was made with an antibody to α-Syn-BAC-Tg/GBA-hetero-KO (Tg) or antibodies to co-stained Erk1/2 active types (pThr202/Tyr204-Erk1 and pThr185/Tyr187-Erk2) of occipital cortex 914 tissues of non-transgenic sibling control (non-Tg) mice of 1, 6, and 24 months old, and an antibody to pSer46-MARCKS. The right graph shows quantitative analysis of pErk1/2 signal intensity in 3 mice (mean value of each mouse, 10 viewing fields). The statistical analysis was carried out by two-way ANOVA, followed by Student's t test. * $p < 0.05$, ** $p < 0.01$.

[Figure 17B] The figure shows activation of Erk1/2 in the cortical neurons of a mouse PD/DLB model and a human DLB patient, more specifically, simultaneous staining of pSer46-MARCKS in the mouse cortex with MAP2 or GFAP.

[Figure 17C] The figure shows activation of Erk1/2 in the cortical neurons of a mouse PD/DLB model and a human DLB patient, more specifically, the same simultaneous staining as above of pErk1/2 and pSer46-MARCKS in the human occipital lobes derived from a DLB patient and a non-DLB control patient (non-DLB patient).

[Figure 17D] The figure shows activation of Erk1/2 in the cortical neurons of a mouse PD/DLB model and a human DLB patient, more specifically, the results of western blot analysis of whole cortex tissues of α-Syn-BAC-Tg/GBA-hetero-KO (Tg) mice or non-transgenic sibling control (non-Tg) mice of 1, 6, and 24 months old, with anti-Pyrk1/2 and - Erk1/2 antibodies.

[Figure 17E] The figure shows activation of Erk1/2 in the cortical neurons of a mouse PD/DLB model and a human DLB patient, more specifically, the results of the same western blot analysis as above of the occipital lobe derived from a human DLB patient.

[Figure 18] The figure shows pSer46-MARCKS in humanized α-Syn-BAC-Tg/GBA-hetero-KO mice of one month old. pSer46-MARCKS and pSer129-α-Syn of normal humanized α-Syn-BAC-Tg/glucocerebrosidase-hetero-KO mice (3 mice per group) were co-stained. The intensity of signal was significantly high in a yellow region.

[Figure 19] The figure shows pSer46-MARCKS in humanized α-Syn-BAC-Tg/GBA-hetero-KO mice of 6 months old. pSer46-MARCKS and pSer129-α-Syn of normal humanized α-Syn-BAC-Tg/glucocerebrosidase-hetero-KO mice (3 mice per group) were co-stained. The intensity of signal was significantly high in a yellow region.

[Figure 20] The figure shows pSer46-MARCKS in humanized α-Syn-BAC-Tg/GBA-hetero-KO mice of 24 months old. pSer46-MARCKS and pSer129-α-Syn of normal humanized α-Syn-BAC-Tg/glucocerebrosidase-hetero-KO mice (3 mice per group) were co-stained. The intensity of signal was significantly high in a yellow region.

[Figure 21] The figure shows comparison of the level of pSer46-MARCKS protein between the peripheral blood cell (PBC) and whole cerebral cortex (brain) of α-Syn-BAC-Tg/GBA-hetero-KO (Tg) mouse of 6 months old or non-transgenic sibling control (non-Tg) mouse.

Description of Embodiments

Now, the present invention will be more specifically described.

The present invention relates to a method for detecting a neurodegenerative disease. In the present invention, detection of a neurodegenerative disease refers to determining that a subject has a neurodegenerative disease or that a subject has a risk of developing a neurodegenerative disease.

The present invention also comprises a method for obtaining ancillary data for detecting a neurodegenerative disease. In the present invention, the neurodegenerative disease includes Alzheimer's disease (AD), frontotemporal lobar degeneration (FTLD), amyotrophic lateral sclerosis (ALS), Parkinson's disease (PD), and dementia with Lewy bodies (DLB).

"Alzheimer's disease" refers to a neurodegenerative disease also called Alzheimer's dementia, and includes "familial Alzheimer's disease" and "hereditary Alzheimer's disease" caused by a gene mutation; and "sporadic Alzheimer's disease" caused by an environmental factor such as a lifestyle habit and a stress.

"Frontotemporal lobar degeneration," which is also referred to as FTLD, refers to a non-Alzheimer's disease type neurodegenerative disease having atrophy in the frontal lobe/temporal lobe in the early stage and whole brain atrophy in the late stage. More specifically, frontotemporal lobar degeneration includes three types of diseases, which are classified based on clinical characteristics, such as frontotemporal dementia (FTD), progressive nonfluent aphasia (PNFA) and semantic dementia (SD); and includes four types of diseases, which are pathologically classified based on the types of proteins accumulated as abnormal proteins in cells, such as FTLD-Tau, FTLD-TDP, FTLD-UPS and FTLD-FUS.

FTLD-Tau is further classified, based on the number of repeats of a microtubule-binding region in a tau protein dominantly accumulated within cells, into 3R Tau type, 4R Tau type and 3/4R Tau type. 3R Tau type includes FTLD (Pick's disease) accompanied by Pick bodies, MAPT (microtubule-related protein tau), and FTLD (FTLD-17) accompanied by a genetic mutation. 4R Tau type includes multi-system tauopathy accompanied by basal ganglia degeneration, progressive supranuclear palsy and dementia, argyrophilic grain dementia (aggressive granular disease), and FTLD (FTLD-17) accompanied by MAPT genetic mutation. 3/4R Tau type includes neurofibrillary dementia and FTLD (FTLD-17) accompanied by MAPT genetic mutation. On the other hand, FTLD group negative to tau and having a ubiquitin-positive inclusion body is called FTLD-U and includes FTLD-TDP, FTLD-UPS and FTLD-FUS as mentioned above.

FTLD-TDP belongs to FTLD-U. Of them, a TDP-43-positive disease is referred to. Examples of the disease include FTLD accompanied by PGRN (progranulin gene) mutation, sporadic FTLD-TDP/FTLD-U, FTLD accompanied by TARDBP (TDP-43 gene) mutation, FTLD accompanied by VCP (valosin-containing protein gene) mutation, and 9 chromosome-linked FTLD. FTLD-FUS belongs to FTLD-U. Of them, a TDP-43-negative and FUS (fused in sarcoma)-positive disease is referred to. Examples of the disease include nerve cell intermediate filament inclusion body disease, atypical FTLD-U, basophilic inclusion body disease, and FTLD accompanied by FUS mutation.

Furthermore, "TLD-UPS" is a type of FTLD-U negative to TDP-43. Examples of the disease include FTLD accompanied by CHMP2B (charged multivesicular protein 2B gene) mutation.

"Amyotrophic lateral sclerosis," which is a type of motor neuron disease, is a neurodegenerative disease characterized by serious muscle atrophy and muscle weakness. As a causative gene of amyotrophic lateral sclerosis, about 20 genes have been reported, and abnormal accumulation of TDP-43 is regarded as a cause.

"Parkinson's disease" is a progressive degenerative disease mainly caused by degeneration of dopamine nerve cells in the substantia nigra. Depigmentation in the substantia nigra/locus coeruleus of the midbrain is observed and nerve cell dropout occurs in the substantia nigra, locus coeruleus, dorsal nucleus of vagus nerve, hypothalamus, and sympathetic ganglion, with the result that shortage of a transmitter substance, dopamine, occurs. Characteristic inclusion bodies called Lewy bodies are observed in the residual nerve cells and part of neurites thereof.

"Dementia with Lewy bodies," which is a disease accompanied by Lewy bodies in the whole cerebral cortex, is degenerative dementia accompanied by not only progressive cognitive impairment but also hallucinations and showing Parkinson's syndrome.

Protein aggregation is a widely observed characteristic in neurodegenerative disorders including Alzheimer's disease (AD), Parkinson's disease, dementia with Lewy bodies, frontotemporal lobar degeneration (FTLD), Huntington's disease (HD), spinocerebellar ataxia (SCA), and amyotrophic lateral sclerosis (ALS). Generally, disease-related proteins presumably take a misfold structure and are later converted into easy-to-aggregate structures including $\beta$-sheet.

However, details of chronological and/or stochastic changes of these structures are rarely known and controversial discussions are aroused as to what is a true toxic species and whether or not aggregated protein or soluble protein is toxic.

Clinical trials for AD treatment have had a lot of influence on these controversial discussions. Passive immunity by an anti-A$\beta$ antibody is certainly successful in reducing the amount of extracellular A$\beta$ plaque in the brain.

However, these clinical trials have reported that there are discrepancies between improvement in A$\beta$-PET (positron emission tomography) and improvement of clinical symptoms.

Accordingly, elucidation of early-stage pathological conditions is an urgent issue for understanding the pathogenesis of AD and development of a treatment. Almost the same circumstances apply to other neurodegenerative diseases including PD/DLB.

Comprehensive phosphoprotein analysis for brain samples taken from mouse AD models and human AD patients elucidated that some proteins start changing, that is, being phosphorylated before extracellular A$\beta$ plaque is histologically detectable (Tagawa et al. (2015) Hum Mol Genet 24:540-558).

Phosphorylation of MARCKS at Ser46 is induced by damage-associated molecular pattern (DAMP) via TLR4, particularly by HMGB1 (Fujita et al, (2016) Sci Rep 6:31895).

This time, similar phosphorylation of MARCKS at Ser46 to that observed in human DLB patients was detected in a BAC-Tg mouse, in which normal human $\alpha$-synuclein ($\alpha$-Syn) of glucocerebrosidase (GBA)-heterojunction knockout (KO) background (human $\alpha$-Syn-BAC-T cells) is excessively expressed. Histological characteristics and chronological progression in the brain of phosphorylation of MARCKS at serine 46 (pSer46-MARCKS) were similar in AD and PD/DLB. Interestingly, this neurodegeneration marker became positive before formation of histologically detectable $\alpha$-Syn aggregates.

These results clearly indicated that neuron degeneration is related with protein aggregation, and that initiation of neurite degeneration precedes formation of protein aggregates.

pSer46-MARCKS can be used as a biomarker for detecting molecular pathology of PD/DLB in the super-early (pre-aggregation/preclinical) stage.

1. Detection of neurodegenerative disease based on the amount of MARCKS phosphorylated at position-46 serine (pSer46-MARCKS)

In the present invention, whether a subject has AD, FTLD or ALS is detected based on phosphorylation of MARCKS used as an indicator in a human biological specimen. The "detect" may be referred to also as "determine" or "evaluate."

MARCKS refers to myristoylated alanine rich protein kinase C substrate and includes a human-derived protein specified by RefSeq ID: NP_002347 (NP_002347.5). Typical examples of a human-derived nucleic acid encoding MARCKS include a nucleic acid containing a coding region (CDS) represented by RefSeq ID: NM_002356 (NM_002356.6).

Examples of the biological specimen to be used as a test specimen include body fluid, a tissue and cells (for example, cerebrospinal fluid, cranial nerve tissue (particularly neurological biopsy tissue), blood, plasma, serum, lymph, urine, saliva) collected from the body of a subject. Of them, cerebrospinal fluid is preferable.

In the present invention, phosphorylation of position-46 serine (Ser) of MARCKS is used as an indicator.

A method for detecting phosphorylated MARCKS is not limited, and any method as far as it is known in the technical field can be used. Examples of the method include mass spectrometry and immunological assay.

The mass spectrometry can be carried out by use of a mass spectrometer. The mass spectrometer comprises a specimen introduction port, an ionization chamber, an analyzing part, a detection part, and a recording part. As the ionization method, matrix-assisted laser desorption ionization (MALDI) or electrospray ionization (ESI) may be used. As the analyzing part, a double focusing mass spectrometer, a quadrupole mass spectrometer (QMS), a time-of-flight mass spectrometry (TOF), a Fourier transform mass spectrometer (FT), and ion cyclotron mass spectrometer (ICR) are used, for example. For accurate analysis, a tandem mass spectrometer (MS/MS) composed of two mass spectrometers connected can be used. The mass spectrometer may be used alone or in connection with, e.g., a separator such as a liquid chromatographic apparatus or a measuring apparatus, or further in combination with a high performance liquid chromatographic apparatus as a liquid chromatograph mass spectrometer (LC/MS, LC/MS/MS).

The immunological assay can be carried out by use of an anti-pSer46-MARCKS antibody, which can recognize MARCKS phosphorylated at position-46 serine (pSer46-MARCKS). Examples of the immunological assay include a solid-phase immunoassay (e.g., RIA, EIA, FIA, CLIA), a dot blotting method, a latex agglutination method (LA: Latex agglutination-turbidimetric immunoassay), and an immunochromatography. An antibody can be immobilized onto a substrate and put in use. Of the immunological assays, enzyme-linked immunosorbent assay (ELISA), which is one of enzyme immunoassay (EIA), is preferred in view of quantitativeness. ELISA is carried out by adding a specimen in antibody-immobilized wells of a microtiter plate, conducting an antigen-antibody reaction, further adding an enzyme labeled antibody, conducting an antigen-antibody reaction, washing, then reacting with an enzyme substrate to develop color, and measuring absorbance. In this manner, a marker protein or a partial peptide thereof in the sample (specimen) is detected; at the same time, the protein or partial peptide concentration in the specimen can be calculated based on the measured value. Furthermore, the antigen-antibody reaction is conducted by use of a fluorescent labeled antibody and then fluorescence may be measured. The antigen-antibody reaction can be carried out at 4°C to 45°C, more preferably 20°C to 40°C, and further preferably 25°C to 38°C. The reaction time is 10 minutes to 18 hours, more preferably 10 minutes to 1 hour, and further preferably 30 minutes to about 1 hour. The antibody to be used in the immunological assay may be a monoclonal antibody or a polyclonal antibody, or a fragment having a binding activity such as Fab, F(ab'), and F(ab')$_2$ of a monoclonal antibody can be used.

The MARCKS phosphorylated at position-46 serine (pSer46-MARCKS) can be detected by positron emission tomography (PET). In the case of detection by PET, a tracer is administered to a subject for imaging pSer46-MARCKS. In this manner, pSer46-MARCKS in the subject may be detected by positron emission tomography (PET). Examples of a tracer for imaging pSer46-MARCKS include an antibody PET tracer prepared by labeling an antibody to a MARCKS protein phosphorylated at position 46 with a positron nuclide.

In the present invention, a specimen collected from a healthy person may be measured as a negative control, at the same time. Herein, "healthy" refers to the condition of a subject having none of neurodegenerative diseases such as Alzheimer's disease (AD), frontotemporal lobar degeneration (FTLD), amyotrophic lateral sclerosis (ALS), Parkinson's disease (PD), and dementia with Lewy bodies (DLB). In this case, if a subject has a neurodegenerative disease such as Alzheimer's disease (AD), frontotemporal lobar degeneration (FTLD), amyotrophic lateral sclerosis (ALS) or Parkinson's disease (PD) and dementia with Lewy bodies (DLB) or has a risk of developing the neurodegenerative disease, the degree of phosphorylation of MARCKS at position-46 serine in the specimen of the subject is higher than that of a healthy person. In other words, the amount of pSer46-MARCKS in the specimen of the subject is higher than that of a healthy person. Accordingly, if the degree of phosphorylation of MARCKS at position-46 serine in the specimen of a subject is higher than that of a healthy person, pSer46-MARCKS is determined as being positive, and it can be determined that a neurodegenerative disease such as Alzheimer's disease (AD), frontotemporal lobar degeneration (FTLD), or amyotrophic lateral sclerosis (ALS) is detected in the subject, in other words, the subject has a neurodegenerative disease such as Alzheimer's disease (AD), frontotemporal lobar degeneration (FTLD), amyotrophic lateral sclerosis (ALS) or Parkinson's disease (PD) and dementia with Lewy bodies (DLB) or has a risk of developing the neurodegenerative disease.

At this time, determination may be made by quantifying pSer46-MARCKS by the aforementioned method and normalizing the obtained quantitative value. Normalization may be made by dividing, for example, the concentration of pSer46-MARCKS by the total concentration of proteins in the specimen. In this case, for example, the amount of pSer46-MARCKS can be expressed by concentration (ppm) relative to the total amount of proteins. Alternatively, pSer46-MARCKS concentration is determined and divided by the total MARCKS (total amount of phosphorylated MARCKS and non-phosphorylated MARCKS) in the specimen. In this case, the amount of pSer46-MARCKS can be expressed by percentage relative to the total amount of MARCKS.

For example, if the pSer46-MARCKS value in the specimen of a subject is 1.3 times or more as large as that in the specimen of a healthy person, preferably 1.5 times or more, further preferably 2.0 times or more, and particularly preferably 3.0 times or more, it can be determined that a neurodegenerative disease such as Alzheimer's disease (AD), frontotemporal lobar degeneration (FTLD), amyotrophic lateral sclerosis (ALS) or Parkinson's disease (PD) and dementia with Lewy bodies (DLB) is detected in the subject.

Alternatively, the amount of pSer46-MARCKS in the specimen of a healthy person may be measured in advance to determine a cutoff value (threshold) based on the measured value of the amount of pSer46-MARCKS. If a measured value exceeds the cutoff value serving as a standard, it is determined that the amount of pSer46-MARCKS in the test specimen collected from a subject is larger than that in the test specimen collected from a healthy person. Consequently, it can be determined that a neurodegenerative disease such as Alzheimer's disease (AD), frontotemporal lobar degeneration (FTLD), amyotrophic lateral sclerosis (ALS) or Parkinson's disease (PD) and dementia with Lewy bodies (DLB) is detected in the subject.

The cutoff value is, for example, set at a mean value of a healthy control group +2 SD. If a measured value exceeds the cutoff value, it can be determined that a neurodegenerative disease such as Alzheimer's disease (AD), frontotemporal lobar degeneration (FTLD), amyotrophic lateral sclerosis (ALS) or Parkinson's disease (PD) and dementia with Lewy bodies (DLB) is detected in the subject.

2. Detection of neurodegenerative disease based on DO (distance from origin) value

In the present invention, using a new parameter, that is, a DO (distance from origin) value, which is obtained by integration of pSer46-MARCKS and non-phosphorylated MARCKS, as an indicator, a neurodegenerative disease such as Alzheimer's disease (AD), frontotemporal lobar degeneration (FTLD), or amyotrophic lateral sclerosis (ALS) is detected. DO is a parameter prepared by integration of the amounts of pSer46-MARCKS and non-phosphorylated MARCKS, in other words, an indicator based on them. When the amount of pSer46-MARCKS and the amount of non-phosphorylated MARCKS protein are plotted on the x-y coordinate system, DO is a value reflecting the distance from the coordinate origin to the plotted point on the coordinate system. In other words, DO is represented by the square root of the value obtained by adding the square of the amount of pSer46-MARCKS and the square of the amount of non-phosphorylated MARCKS, more specifically, expressed by the following formula:

[Formula 1]

$$DO = \sqrt{(pSer46\text{-}MARCKS)^2 + (non\text{-}phosphorylated\text{-}MARCKS)^2}$$

where "pSer46-MARCKS" represents the amount of a MARCKS protein phosphorylated at position 46; and "non-phosphorylated-MARCKS" represents the amount of a non-phosphorylated MARCKS protein.

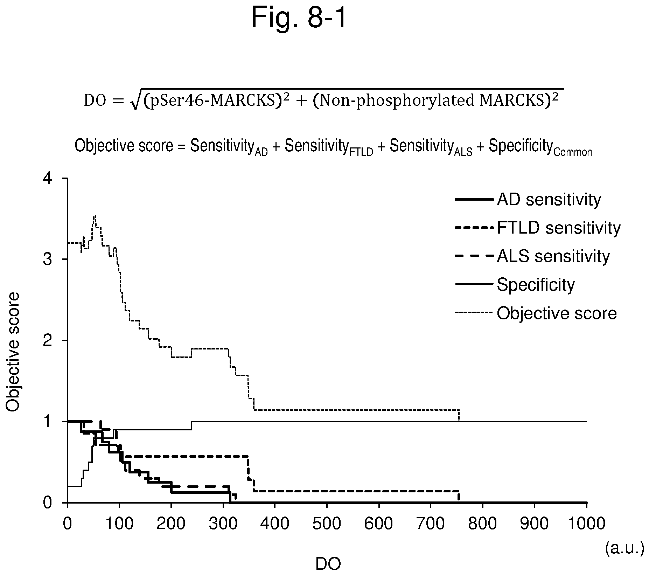

If the DO value is used, compared to the case where pSer46-MARCKS alone is used as an indicator, a neurodegenerative disease such as Alzheimer's disease (AD), frontotemporal lobar degeneration (FTLD), or amyotrophic lateral sclerosis (ALS) can be detected with a higher sensitivity and a higher specificity. If numerical simulation of the DO value is carried out, as shown in Figure 8-1, it is possible to determine an upper limit (cutoff value) of a normal (DO) value at which satisfactory sensitivity and specificity are obtained. As shown in the numerical simulation of Figure 8-1, using another parameter, called "objective score," which is prepared by adding up the sensitivity of three diseases, i.e., Alzheimer's disease (AD), frontotemporal lobar degeneration (FTLD), and amyotrophic lateral sclerosis (ALS) (maximum = 1.0 in each disease, that is, maximum = 3.0 in three diseases) and specificity common in the three diseases (maximum = 1.0, as a representative value of three diseases), the range of DO providing the highest objective score may be selected. For example, if normalized values by total protein concentration in the specimen are used as the amounts of pSer46-MARCKS and non-phosphorylated MARCKS, the cutoff value of the DO value becomes 50 to 55 (ppm); the sensitivity becomes 0.8 to 1.0; and the specificity becomes 0.8 or more, preferably 0.8.

Alternatively, DO value with respect to a healthy person is calculated by previously measuring the amounts of pSer46-MARCKS and non-phosphorylated MARCKS in a specimen of the healthy person and the DO cutoff value (threshold) may be determined based on the calculated value. If a DO value exceeds the cutoff value serving as a standard, it can be determined that a neurodegenerative disease such as Alzheimer's disease (AD), frontotemporal lobar degeneration (FTLD), or amyotrophic lateral sclerosis (ALS) is detected in a subject, in other words, the subject has a neurodegenerative disease or a risk of developing a neurodegenerative disease.

In Alzheimer's disease (AD) and frontotemporal lobar degeneration (FTLD), seriousness of the disease is associated with the DO value. That is, it can be determined that the higher the DO value, the more serious the disease.

The level of pSer46-MARCKS in the brain tissue increases also in Parkinson's disease (PD) and dementia with Lewy bodies (DLB), as in Alzheimer's disease (AD) and frontotemporal lobar degeneration (FTLD). Thus, also in Parkinson's disease (PD) and dementia with Lewy bodies (DLB), neurodegeneration can be detected by checking an increase of pSer46-MARCKS level in a specimen and pSer46-MARCKS level can reflect severity.

3. Treatment for neurodegenerative disease

In the case where a neurodegenerative disease is detected by the method of the present invention, Alzheimer's disease (AD), frontotemporal lobar degeneration (FTLD), amyotrophic lateral sclerosis (ALS), Parkinson's disease (PD), or dementia with Lewy bodies (DLB) may be treated or the symptom of the disease may be improved by the following method.

Alzheimer's disease (AD)

The disease is treated by, e.g., administering a medical drug for promoting signal transduction between neurons, such as donepezil, rivastigmine, galantamine, and memantine, and administering an antibody drug, which can remove amyloid β, a causative substance of Alzheimer's disease, from the brain, such as bapineuzumab and solanezumab.

Frontotemporal lobar degeneration (FTLD)

The disease is treated by administering a selective serotonin reuptake inhibitor and a therapy targeting a causative gene such as tau gene, TDP-43 gene, and progranulin gene.

Amyotrophic lateral sclerosis (ALS)

The disease is treated by, e.g., administering a medical drug such as riluzole, edaravone, methylcobalamin, retigabine, BIIB067 and MN-166, a therapy targeting a gene such as SOD1 gene, TDP-43 gene, FUS gene, and C9orf72 gene, which are reported as a causative gene, and a regenerative therapy for regenerating nerve cells by use of human pluripotent stem cells.

Parkinson's disease (PD)

The disease is treated by a drug treatment, a surgical treatment, and a physical therapy. As the drug treatment, internal use of L-dopa and a dopamine agonist is known. As the surgical treatment, a deep brain stimulation therapy (subthalamic nucleus stimulation, pallidal stimulation, thalamic stimulation) and stereotactic destruction (thalamus destruction, pale globe destruction) or a deep brain stimulation therapy (DBS) is known.

Dementia with Lewy bodies (DLB)

The disease is treated by administrating a medical drug such as donepezil.

Examples

The present invention will be more specifically described by way of the following Examples; however, the present invention is not limited by these Examples.

[Example 1] Study on Alzheimer's disease (AD), frontotemporal lobar degeneration (FTLD), and amyotrophic lateral sclerosis (ALS)

Methods

Human patients

Cerebrospinal fluids were taken from 8 AD patients, 7 FTLD patients, and 10 ALS patients who were diagnosed in Nagoya University and Tohoku University, based on clinical symptoms, electrophysiological examination, and neuroimaging including MRI, SPECT, and PET, and put in use.
Control subjects were 6 males (55 to 83 years old, average age: 72.6) and 4 females (69 to 79 years old, average age: 75.5). The scores of the mini mental-state examination (MMSE) of them when the cerebrospinal fluid (CSF) was collected were 25 or more (26 to 30, average: 28.3). AD patients were 2 males (54 and 80 years old) and 6 females (57 to 75 years old, average: 64.5 years old). The MMSE scores of these patients were not more than 25 (4 to 25, average: 17.1) and the average and range of the FAB scores were 9.3 and 6 to 13, respectively. FTLD patients were 5 males and 2 females. The average and range of the MMSE scores of these patients were 18.1 and 7 to 30, respectively; and the average and range of FAB scores were 8.6 and 3 to 16, respectively. ALS patients were 8 males and 2 females. The average and range of ALSFR-R scores of these patients were 40.5 and 24 to 47, respectively. 9 ALS cases were "confirmed" diagnosis and a single case was diagnosed as "possible" of ALS.

Preparation of sample

Specimens for proteome analysis were prepared from the cerebrospinal fluids (CSFs). More specifically, 50 $\mu$l of the cerebrospinal fluid was taken from a human patient, added in 5 $\mu$l of a buffer containing 100 mM Tris-HCl (pH 7.5), 2% SDS, and 1 mM DTT and incubated at 100°C for 15 minutes.

The sample solution was centrifuged at 16,000 g and 4°C for 10 minutes. The supernatant was filtered by use of 0.22 $\mu$m PVDF filter (Millipore Corporation). An aliquot (55 $\mu$L) was taken, added to 25 $\mu$L of 1 M triethylammonium bicarbonate (TEAB) (pH 8.5), 0.75 $\mu$L of 10% SDS, and 7.5 $\mu$L of 50 mM tris-2-carboxyethyl phosphine, and incubated at 60°C for 1 hour. Cysteine residues were blocked by 10 mM methyl methanethiosulfonate for 10 minutes at 25°C. Then, the sample was digested with 1.5 $\mu$g of trypsin in 24 mM $CaCl_2$ (protein:enzyme = 10:1 w/w) at 37°C for 24 hours, desalted by use of C18 spin column (MonoSpin C18, GL Sciences Inc.), dried and dissolved in 35 $\mu$l of 0.1% formic acid.

Mass spectrometry of SWATH-cerebrospinal fluid

30 $\mu$l of an aliquot was analyzed in C18 column (0.1 mm $\times$ 100 mm, KYA Technologies Corporation) containing solution A (0.1% formic acid) in a gradient of 2 to 41% solution A and B (99.9% acetonitrile and 0.1% formic acid) at a flow rate of 300 nl/min by Eksigent NanoLC-Ultra 1D Plus system (Sciex Inc.) and subjected to Triple TOF 5600 system (Sciex) at an ion spray voltage of 2.3 kV. Information-dependent acquisition (IDA) was carried out by setting at 400 to 1000 m/z through application of electric charge 2 to 5 times. The MS/MS scan of generated ions was carried out in the range of 100 to 1600 Da. The accumulation time of a spectral library was 100 ms. SWATH (registered trademark) (sequential window acquisition of all theoretical mass spectra) was carried out by 24 sequential windows of 25 Da from 400 Da to 1000 Da. SWATH of MS/MS spectral data was carried out by Analyst TF1.6 software (Sciex Inc.) at 100 ms/window and MS/MS spectral library was prepared by Protein Pilot software (version 4.5). MS/MS spectral data were made correlation with peptide data and LC retention time by use of Peakview software (version 1.2.0.3, Sciex Inc.). The amounts of MS/MS products (ions) from the same peptide were summed up and used as the amount of the peptide.

Mass data analysis

Using SWATH 1.0 application of PeavView1.2 software, MS/MS spectrum raw data were processed under the conditions of 5 minutes of ion chromatogram (XIC) extraction window and XIC width of 0.01 Da. In this manner, the spectrum of a species clearly identified was extracted. XIC was indicated as a curve in a graph showing LC retention time versus relative ionic strength within a narrow m/z range. Fragmented ion XICs were summed up to obtain a peptide peak region, and a plurality of peptide areas per protein were summed up to obtain a protein area. Mass spectrum data of a peptide were normalized by total amount of all proteins detected per subject or the total amount of the peptides correlated with MARCKS protein (these are referred to respectively as "total protein" or "total MARCKS"). In order to calculate sensitivity and specificity, abnormal phosphorylation was determined based on the average amount $\pm2$ SD of the control group.

Immunohistochemical analysis

For immunohistochemical analysis, human brain sections embedded in paraffin were deparaffinized, rehydrated and irradiated with microwave five times in a 10 mM citrate buffer (pH 6.0, 100°C), and then, washed twice in PBS for 5 minutes. After blocked (10% FBS, 60 minutes), the sections were incubated in PBS containing 2% FBS and 0.1% Triton X-100 together with a primary antibody: rabbit anti-phospho-TDP43 (pS409/410-2) (1:4000, Cosmo Bio Co., LTD, TIP-PTD-P02), rabbit anti-phospho-MARCKS (Ser46) (1:1000, GL Biochem (Shanghai) Ltd.), and mouse anti-MAP2 (AP20) (1:100, Santa Cruz Biotechnology, Inc.) at 4°C overnight or for 2 nights, and finally with a secondary antibody: Alexa Fluor 488 labeled anti-mouse IgG (1:1000, Invitrogen Corporation), Cy3 labeled anti-rabbit IgG (1:500, Jackson ImmunoResearch Laboratories, Inc.), biotinylated anti-rabbit IgG (1:200, Vector Laboratories, BA-10009), and biotinylated anti-mouse IgG (1:200, Vector Laboratories, BA-2000) at room temperature (RT) for 60 minutes. In this manner, a pSer46-MARCKS antibody was produced. The same primary antibodies derived from the host species (rabbit anti-phospho-MARCKS and anti-phospho-TDP43 antibody) was labeled by use of Zenon Rabbit IgG Labeling Kits (Thermo Fisher Scientific, IL) and used in double immunostaining. For enzymatic detection of phospho-TDP43 or MAP2, VECTASTAIN Elite ABC standard kit (Vector Laboratories, PK-6100 CA) and DAB peroxidase substrate kit (Vector Laboratories, SK-4100) were used. In the case of DAB staining, the sections were counterstained by use of Carrazzi's Hematoxylin solution (1.15938.0025, Merck KGaA) at room temperature for 10 minutes, washed with tap water for 10 minutes, stained in an eosin solution (0.25% eosin diluted with 80% ethanol, Wako) at room temperature for 5 minutes and dewatered with ethanol. After washed with xylene, the sections were covered. The nuclei were stained with DAPI (Dojindo Laboratories, D523). Images were produced by a confocal microscope (Olympus Corporation, FV1200 IX83) and an optical microscope (Olympus Corporation, BX53).

Nissl staining

Human brain sections embedded in paraffin were deparaffinized and rehydrated. After washed with distilled water, the sections were soaked in a cresyl violet solution (300 mL of 0.1% cresyl violet was added to distilled water together with 5 droplets of a 10% acetic acid solution and then filtered before use) at 37°C for 15 minutes. Then, the sections were dewatered with ethanol, and washed with xylene and a cover glass was placed on the section. Images were produced by an optical microscope (Olympus Corporation, BX53).

Numerical simulation

As a parameter for maximization by numerical simulation of the upper limit of a normal case, sensitivity values from three diseases (maximum = 1.0 in each disease; maximum = 3.0 in three diseases) and the total of specificity values of three diseases, the specificity value being common in three diseases (maximum = 1.0, as a representative of three diseases) were used. Furthermore, a numerical simulation program using R programming language (version 3.4.0, R statistics computing, https://www.R-project.org/) was originally developed, and the upper limit of a normal case maximizing the parameter was optimized. DO value and a main part of its category were input. In the first trial, the program was executed in order to determine an essential analysis range. The upper limit of DO of the simulation when the sensitivity scores of three diseases became 0 was determined as 1,000. Within the range, numerical simulation was repeated to find optimal DO which maximizes an objective score.

Statistical analysis

The biological distance between the disease group and the control group was tested by the Wilcoxon rank sum test. To evaluate the correlation between pSer46 and a non-phosphorylated peptide per subject, the Pearson correlation coefficient was calculated. Using the Pearson correlation coefficient, the correlation between HMGB1 concentration and pSer46-MARCKS signal per subject was also calculated.

Results

pSer46-MARCKS in the cerebrospinal fluid (CSF) of a neurodegenerative disease

Using mass spectrometry, pSer46-MARCKS in the cerebrospinal fluids (CSFs) taken from human patients with three neurodegenerative diseases, Alzheimer's disease (AD), frontotemporal lobar degeneration (FTLD), and amyotrophic lateral sclerosis (ALS) was quantified, simultaneously with pSer46-MARCKS of a control. In order to compare the relative amounts of the phosphorylated peptides of disease groups or individual subjects, the amounts of individual subjects were normalized by the total amount of proteins or total MARCKS proteins detected by mass spectrometry. The peptide was determined with a confidence of 95% or more.

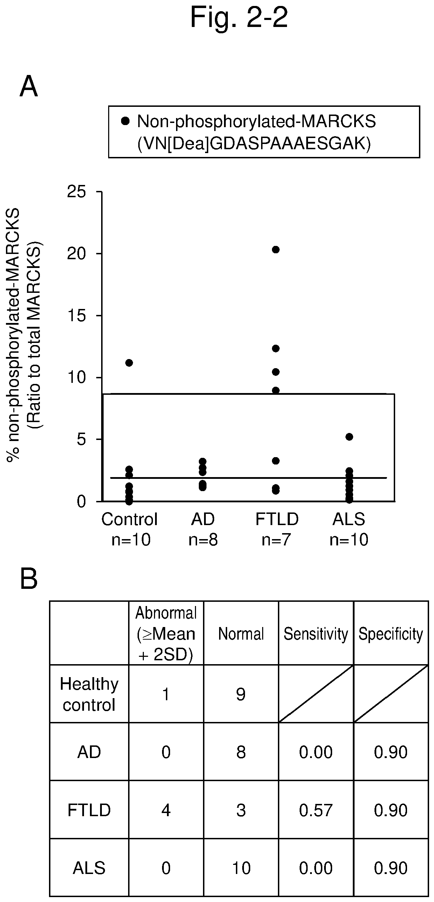

In order to evaluate pSer46-MARCKS in the cerebrospinal fluid (CSF) as a biomarker for diagnosis, the sensitivity and specificity of pSer46-MARCKS normalized by the total protein (Figures 1-1A and B) or total MARCKS protein (Figures 1-2A and B), in the three disease groups, were calculated. Figure 1-1A is a graph obtained by plotting values of pSer46-MARCKS separately by disease group. The values were previously normalized by the total amount of proteins of individual subjects. Figure 1-2A is a graph obtained by plotting values of pSer46-MARCKS separately by disease group. The values were previously normalized by the total amount of MARCKS proteins of individual subjects. The rectangular box in the lower part of each of Figure 1-1A and Figure 1-2A indicates a mean number of the control group $\pm 2$ SD and the line in the box indicates a mean of the control group (group of healthy subjects). Figures 1-1B and 1-2B each show sensitivity and specificity. When the value of a patient was beyond the mean of the control group +2 SD, abnormality was determined. In this state, it was found that the specificity of pSer46-MARCKS in all three groups with neurodegenerative diseases, is extremely high (Figure 1-1B and Figure 1-2B). This means that if pSer46-MARCKS is beyond the mean of the control +2 SD, the subject is definitely abnormal.

In contrast, the sensitivity of pSer46-MARCKS for diagnosis was relatively low in all disease groups. In the case where pSer46-MARCKS was normalized by the total protein, the sensitivity of AD was 0.50, FTLD, 0.57 and ALS, 0.40 (Figure 1-1B). In the case where pSer46-MARCKS was normalized by total MARCKS, the value was smaller (Figure 1-2B). Accordingly, in this analysis stage, it was considered appropriate that use of pSer46-MARCKS is limited just like a progressive biomarker not like a disease-specific biomarker or a screening biomarker.

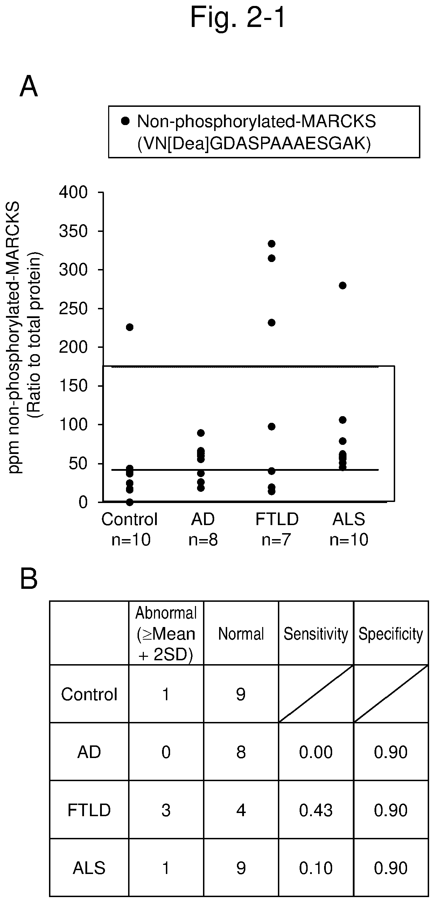

Non-phosphorylated MARCKS in the cerebrospinal fluids (CSFs) taken from human patients with three neurodegenerative diseases (Figures 2-1 and 2-2) were quantified in the same manner as above. Figure 2-1A is a graph obtained by plotting the amounts of non-phosphorylated MARCKS of individual disease groups. The values were previously normalized by the total protein. Figure 2-2A is a graph obtained by plotting the amounts of non-phosphorylated MARCKS of individual disease groups. The values were previously normalized by the total amount of MARCKS proteins. The rectangular box in the lower part of each of Figure 2-1A and Figure 2-2A indicates a mean number of the control group $\pm 2$ SD and the line in the box indicates a mean of the control group (group of healthy subjects). Figures 2-1B and 2-2B each show sensitivity and specificity. In this case, specificity in AD and ALS did not reach 100% and sensitivity was 0. Accordingly, it was apparent that non-phosphorylated MARCKS alone is not suitable for a diagnostic biomarker.

Increase of pSer46-MARCKS in the cerebrospinal fluid (CSF) of AD, FTLD and ALS

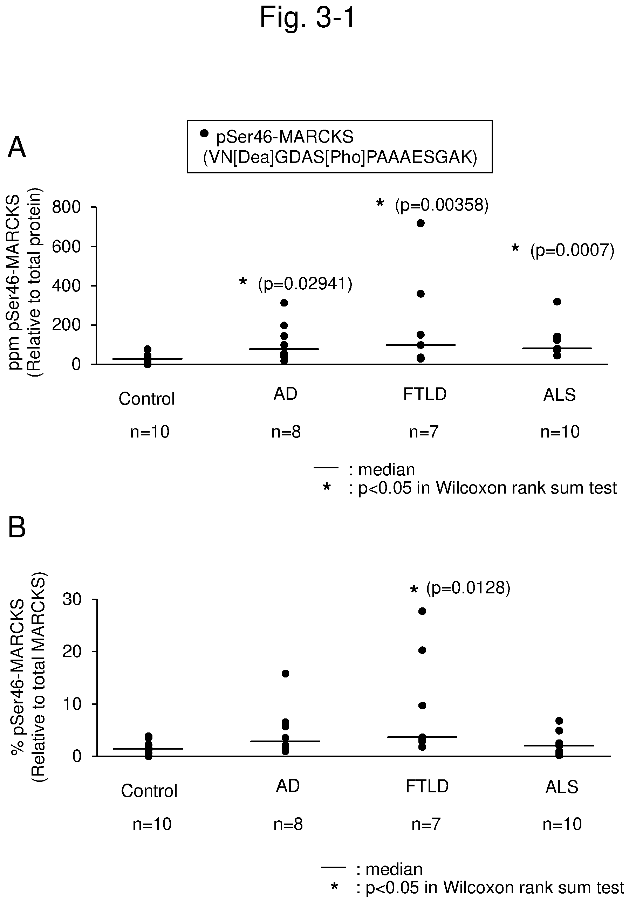

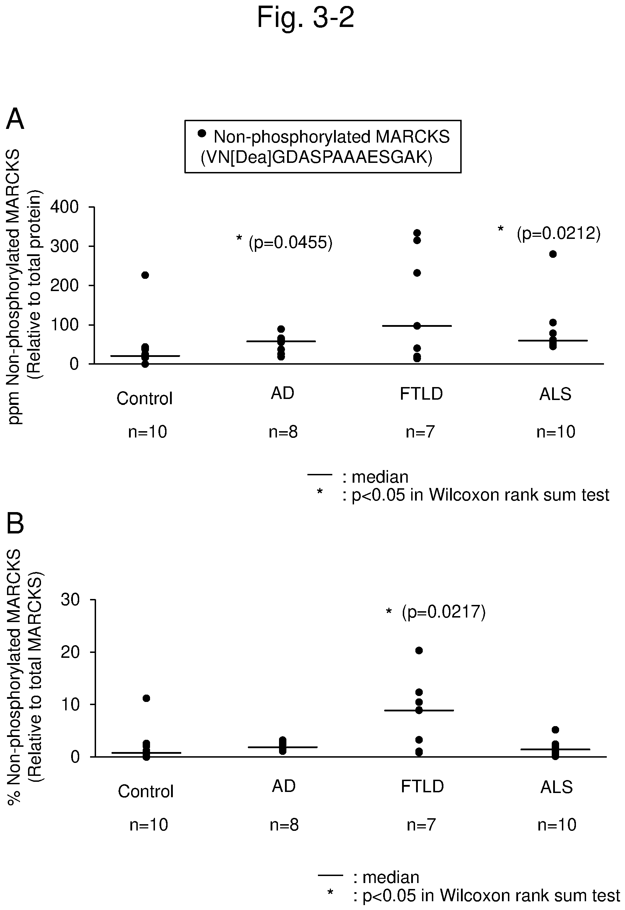

The value of pSer46-MARCKS was compared between the disease groups and the control. Figure 3-1 shows the amounts of pSer46-MARCKS of individual subjects and Figure 3-2 shows the amounts of non-phosphorylated MARCKS of individual subjects. The amounts in Figures 3-1A and 3-2A were normalized by the total amount of proteins. The amounts in Figures 3-1B and 3-2B were normalized by the total amount of MARCKS. The lines in the figures show medians of each group. In order to compare values of individual groups in consideration of the case where a value equal to or higher than the values observed in the disease groups may present, the non-parametric Wilcoxon rank sum test was carried out. As a result, it was found that there are statistical differences between AD patients and the control (p = 0.02941), FTLD and the control (p = 0.00358), and ALS and the control (p = 0.0007) (Figure 3-1A). The results changed by normalization methods for pSer46-MARCKS. When pSer46-MARCKS was normalized by total MARCKS (Figure 3-1B), the difference between the FTLD patients and the control (p = 0.0128) was maintained. Further, in the case where the values of non-phosphorylated MARCKS were normalized by the total protein, it was found that statistical difference is present between AD patients and the control (p = 0.0455) and between ALS patients and the control (p = 0.0212) (Figure 3-2A). Further, in the case where the values were normalized by total MARCS, it was found that statistical difference is present between FTLD patients and the control (p = 0.0217) (Figure 3-2B).

Relationship between pSer46-MARCKS and non-phosphorylated MARCKS

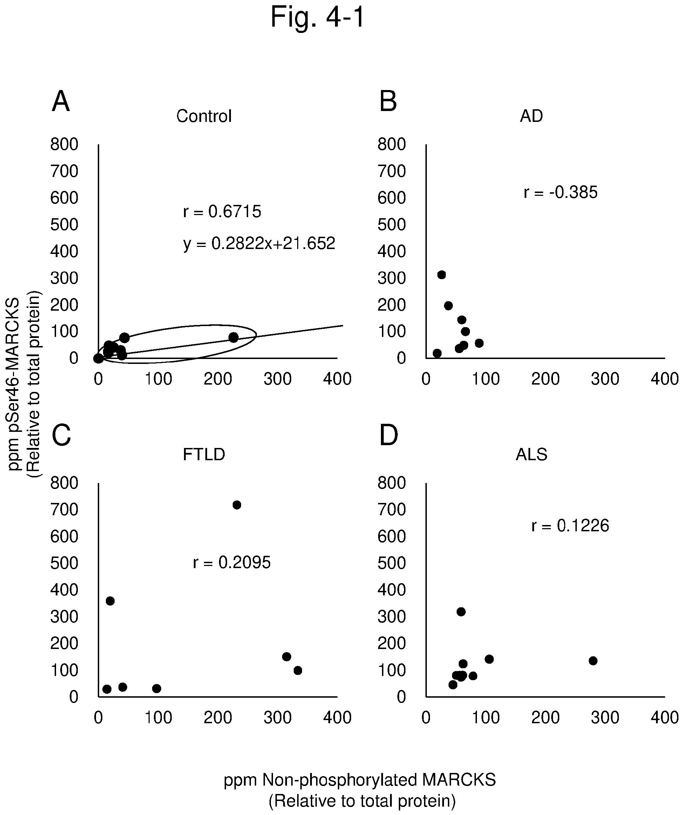

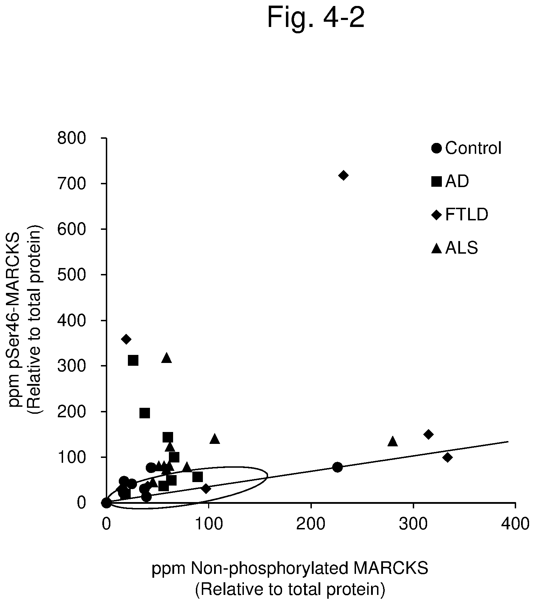

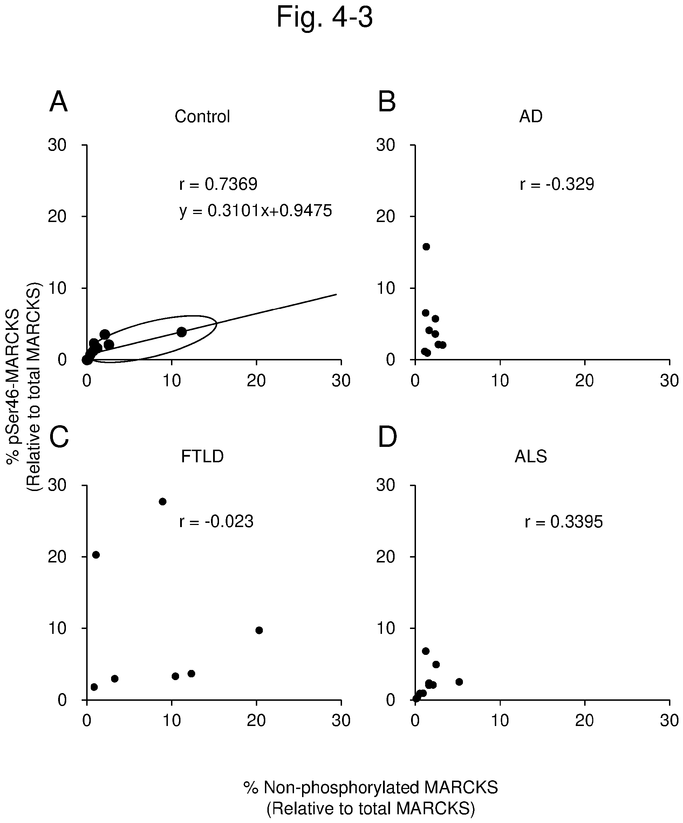

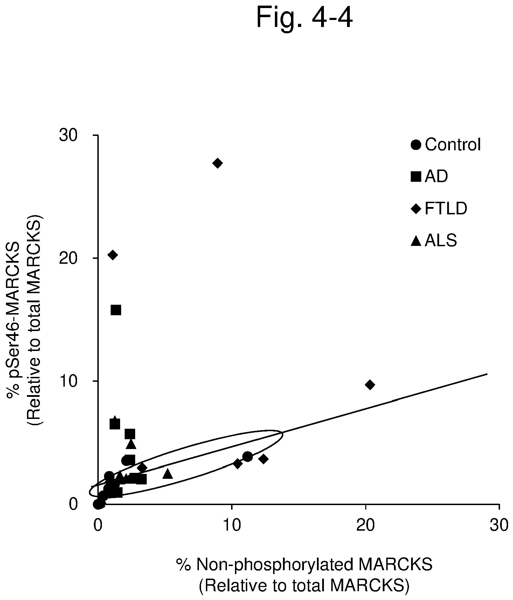

The relationship (ratio) between pSer46-MARCKS and non-phosphorylated MARCKS in each of the groups was analyzed (Figures 4-1 to 4-3). Figure 4-1 is a graph obtained by plotting the values of AD, FTLD and ALS disease groups, which were normalized by the total amount of proteins. Figure 4-2 is a graph obtained by merging plots of individual groups shown in Figure 4-1. Figure 4-3 is a graph obtained by plotting the values of AD, FTLD and ALS disease groups, which were normalized by the total amount of MARCKS. Figure 4-4 is a graph obtained by merging plots of individual groups shown in Figure 4-3. In the control group, there was a clear positive relationship between pSer46-MARCKS and non-phosphorylated MARCKS (Figures 4-1 and 4-3); however, a weak negative relationship was shown in AD group, and none of relationships was shown in FTLD and ALS groups. The results could mean that phosphorylation of MARCKS at Ser46 is pathologically influenced in AD and FTLD.
Another point that should be noted was that plots of the disease groups are mostly positioned on the regression line on the coordinate system (x, y = non-phosphorylated MARCKS, pSer46-MARCKS), which was determined by the control group (Figures 4-2 and 4-4). This means that, regardless of whether the total protein or total MARCKS was used for normalization of pSer46-MARCKS (Figures 4-1 to 4-4), the ratio of pSer46-MARCKS to non-phosphorylated MARCKS tends to increase in AD, FTLD and ALS patients. Based on these analyses, whether the ratio of pSer46-MARCKS to non-phosphorylated MARCKS can be used as a biomarker for pathological changes was examined. However, the difference between the groups did not have statistical significance (Figures 4-1 to 4-4). A big difference in score (Figures 4-1 to 4-4) is probably derived from heterogeneity of patient groups in which patients of early and late disease phases were present together.

Relationship between HMGB1 and pSer46-MARCKS in the cerebrospinal fluid (CSF)

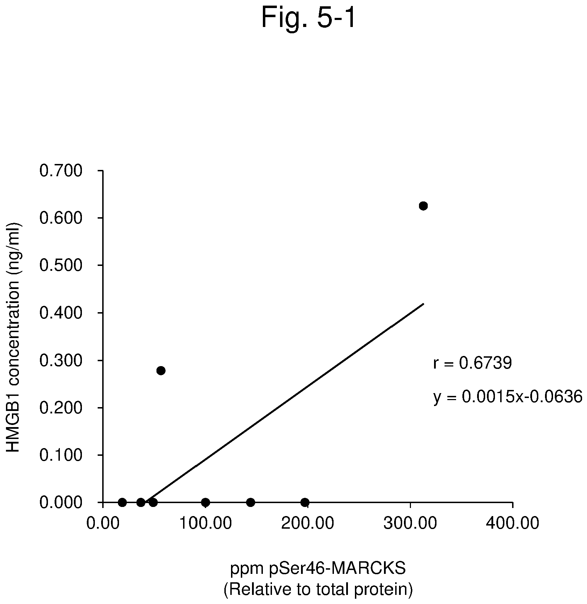

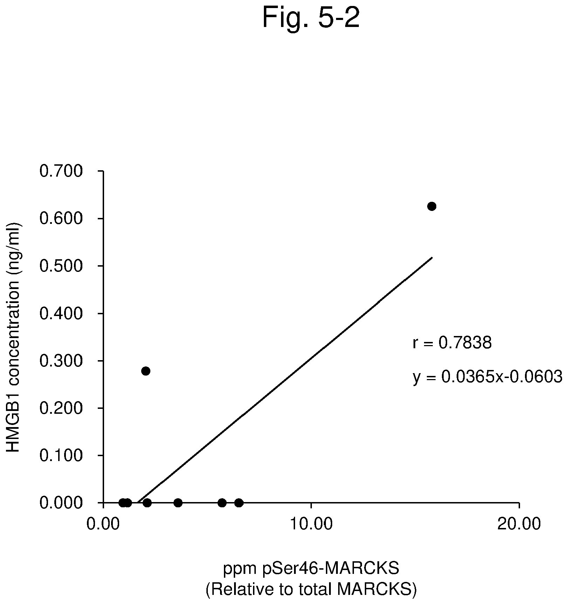

The present inventors previously revealed the possibility that HMGB1, which is typically known as a molecule belonging to damage-associated molecular patterns (DAMPs), induces phosphorylation of MARCKS at Ser46 downstream of the signal transduction pathway of TLR4, with the result that an antibody to HMGB1 delays onset of AD13. Based on a series of ideas and data previously obtained, the relationship between HMGB1 and pSer46-MARCKS in the cerebrospinal fluids (CSFs) of AD patients was examined (Figures 5-1 and 5-2). Figure 5-1 shows pSer46-MARCKS relative to the total amount of proteins; and Figure 5-2 shows pSer46-MARCKS relative to the total amount of MARCKS. To find that the hypothesis about HMGB1 and pSer46-MARCKS can be actually applied *in vivo,* retrospective analysis based on data previously reported (by another tester) must be carried out by a non-bias test.
There is a technical limit in HMGB 1-ELISA sensitivity, that is, threshold. The HMGB1 values were a detection level or less in almost all patients, but a positive correlation higher than expected was clearly found between the cerebrospinal fluid (CSF) of AD patients and PSER46-MARCKS (Figures 5-1 and 5-2).

Increase of pSer46-MARCKS in dystrophic neurites of human FTLD

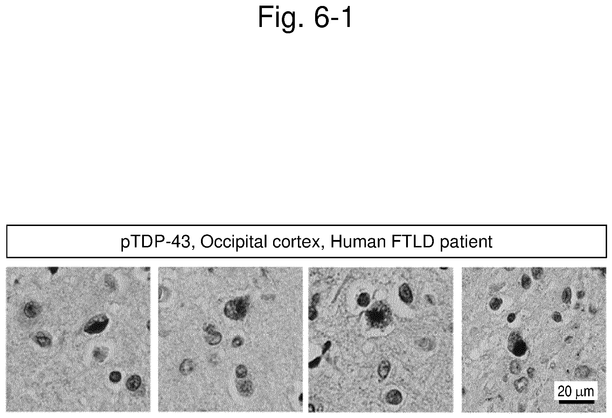

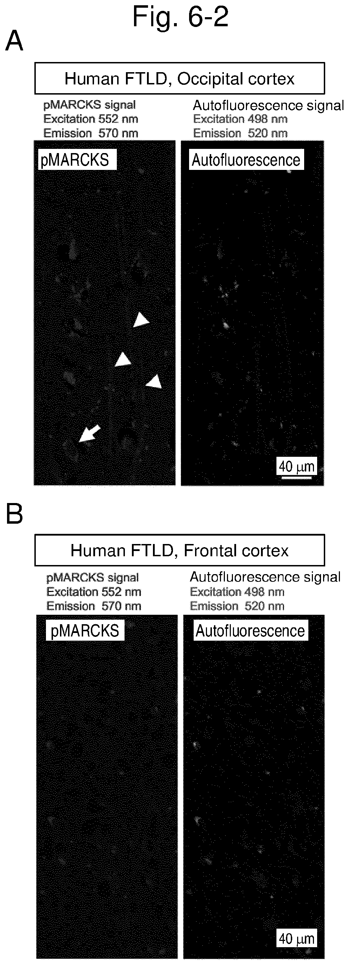

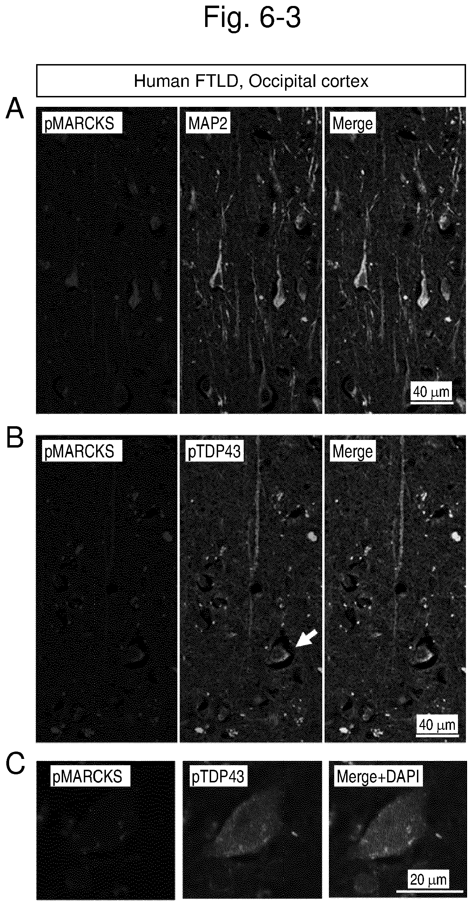

In AD mouse models and AD human patients, whether pSer46-MARCKS increased or not was examined in dystrophic neurites of human FTLD brain (Fujita, K. et al., Sci. Rep. 6, 31895 (2016)). Human FTLD brain (Figure 6-1) pathologically diagnosed was subjected to immunohistochemical analysis using an anti-pSer46-MARCKS antibody (Figures 6-2A and B). Figure 6-1 shows a stained image of pTDP-43 in human FTLD. By staining of TDP43, abnormal distribution of TDP43 and enclosure of TDP43 within the cytoplasm were found. Furthermore, Figures 6-2A and B show immunohistochemical analysis by an anti-pSer46-MARCKS antibody. As shown in Figure 6-2A, neurite degeneration was observed in the FTLD occipital lobe (occipital cortex) and frontal lobe (frontal cortex) (the IVth layer). Furthermore, the dendrite of the occipital lobe apex and cell bodies were well stained. However, as shown in Figure 6-2B, the frontal lobe, which was most seriously affected region, was almost not stained. After homogeneous staining was carried out by using an anti-pSer46-MARCKS antibody and a secondary Cy3 labeled anti-rabbit IgG antibody, the frontal cortex was visible at an excitation light wavelength of 552 nm and at a fluorescence wavelength of 570 nm but not visible at an excitation light wavelength of 498 nm and at a fluorescence wavelength of 520 nm (Figure 6-2B) or at an excitation light wavelength of 552 nm and at a fluorescence wavelength of 520 nm (data not shown). This demonstrates that staining is specific and autofluorescence did not occur. A staining pattern was extremely analogous to that of human AD shown in a previous report (Fujita, K. et al., Sci. Rep. 6, 31895 (2016)). In FTLD, in the occipital lobe relatively less affected, pSer46-MARCKS-positive neurite was frequently observed but less frequently observed in the frontal lobe most strongly affected. This controversial finding shows that pSer46-MARCKS is an early-stage specific marker and disappears as the disease stage is advanced. Figure 6-3A shows the results of co-staining of MAP2 and pSer46-MARCKS in human FTLD occipital lobe. Neurons were stained with an anti-pSer46-MARCKS antibody. In the human FTLD brain, dendrites and cell bodies stained with pSer46-MARCKS are also positive to MAP2. From this, it is shown that these are neurons and neurites (Figure 6-3A). Subsequently, it was confirmed that the pSer46-MARCKS-positive cells exhibit abnormal distribution of neurons having aggregates or TDP43 in the cytoplasm. More specifically, Figure 6-3B shows that neurons having phosphorylated TDP43 in the cytoplasm are co-stained together with pSer46-MARCK. Interestingly, pSer46-MARCKS and phosphorylated TDP43 were co-stained at cytoplasm aggregates (Figure 6-3C).

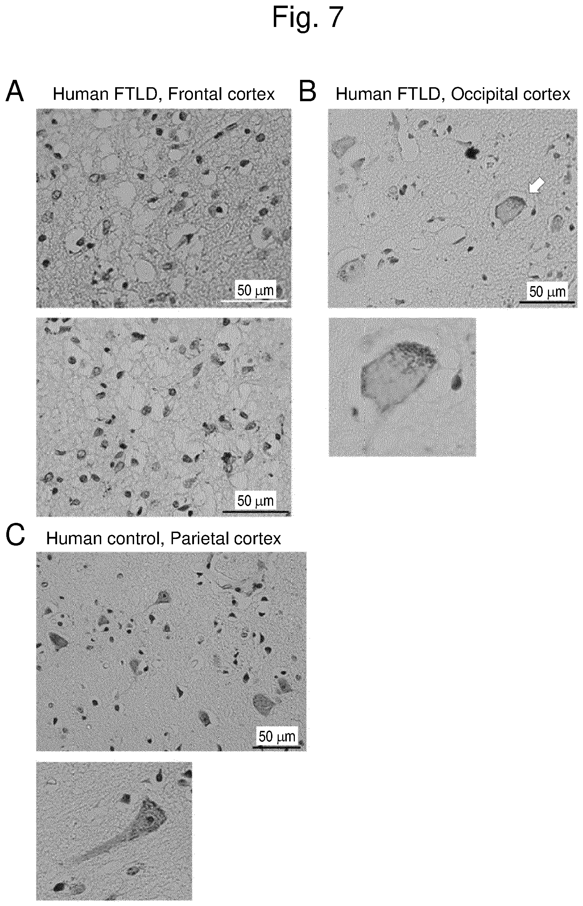

In the staining of human FTLD with Nissl, neurons of the frontal lobe were not substantially detected and only sponge-like degeneration was found in vacuole mass (Figure 7A). In contrast, in the occipital lobe, neurons having abnormal features, such as chromatolysis and achromasia, were observed (Figure 7B). In the brain of the control having no neurodegenerative disease, such abnormal changes with respect to neuron density and neural network form were not observed (Figure 7C).

Integration value of pSer46-MARCKS and non-phosphorylated MARCKS as a biomarker

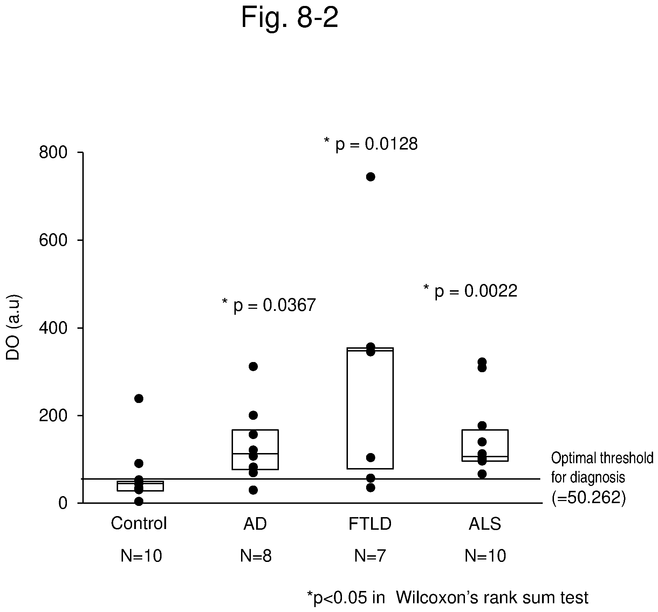

From graphs (Figure 4-2 and 4-4), it is found that the position (x, y) of a plot of a control subject in the coordinate system is relatively close to the origin (0,0). Accordingly, it was predicted that a pathological change may be distinguished from a normal control by the degree of change in integration of pSer46-MARCKS and non-phosphorylated MARCKS. Then, DO (distance from origin), which was obtained by the formula: a square root of ($[pSer46\text{-}MARCKS]^2$ + $[non\text{-}phosphorylated\ MARCKS]^2$), was calculated with respect to a control case and a disease case (Figure 8-1). As a result, the DO value statistically increased in three neurodegenerative disease groups, compared to the control group. Significant difference between them was confirmed by Wilcoxson test (Figure 8-2). However, there was an exception in the control group, that is, a single subject exhibited a large DO value. The subject exhibited a sharp decrease in ADAS score (-3.216949153/year). From the fact, it is determined that the subject might have been in the preclinical stage of dementia.

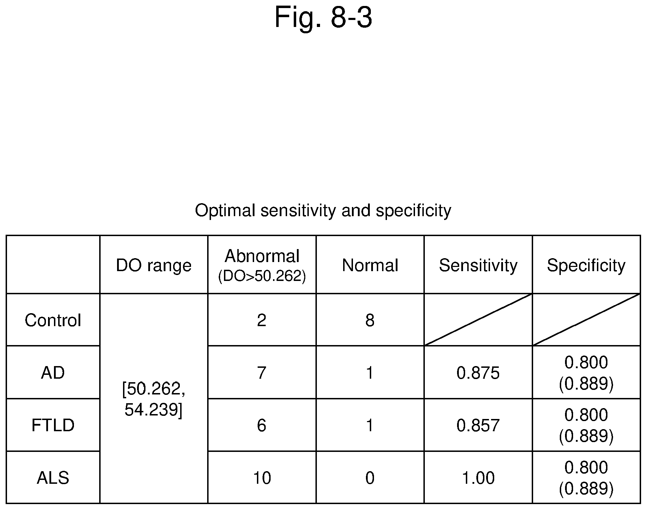

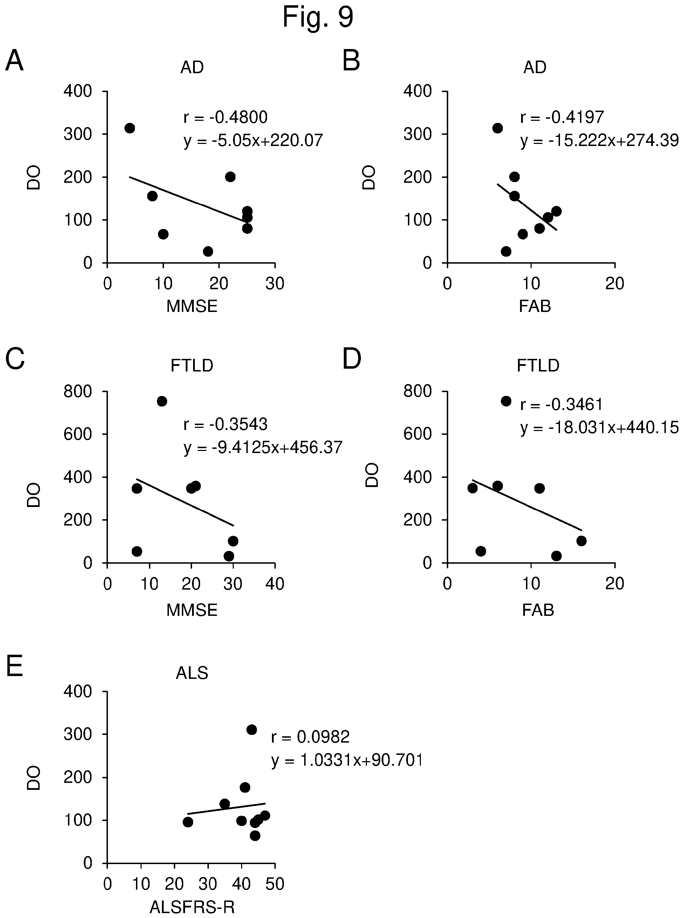

To efficiently distinguish a pathological DO value from a normal DO value, "the upper limit of normal DO," which can provide both maximum sensitivity and specificity, must be determined. The ideal "upper limit of normal DO" was searched by numerical simulation (Figure 8-1). Herein, another parameter, i.e., "objective score" was used, which is a total of sensitivity of three diseases (maximum = 1.0 in each disease, that is, maximum = 3.0 in three diseases) and specificity common in the three diseases (maximum = 1.0, as a representative of three diseases). The inventors tried to find the range of DO providing the highest objective score (Figure 8-1). The graph of Figure 8-1 shows the objective score versus DO in the range of 0 to 1000. As a result, it was found that the highest objective score of 3.532 can be obtained at the upper limit value of DO within the range of 50.26 to 54.24 (Figures 8-1 and 8-2). Figure 8-2 shows DO values of individual diseases. In the figure, DO values are shown by plots within a box. The upper limit of a normal case was determined by numerical simulation. The values from the lower limit to the upper limit of each group are enclosed by the box. The median value is also within the box. Figure 8-3 shows the number of cases determined as being abnormal in control, AD, FTLD and ALS groups, target scores, and values of sensitivity and specificity at the maximum optimization time in the DO range. The threshold in Figure 8-1 was temporarily represented by DO of 50.262; however sensitivity and specificity did not change within the DO range from 50.262 to 54.239. As shown in Figure 8-3, if the upper-limit normal value was determined as 50.26, it was possible to determine abnormal values with a sensitivity of 0.875, 0.857, and 1.00 in AD, FTLD, and ALS, respectively (Figure 8-3, Figure 9). The specificity was 0.800 in all disease cases (Figure 8-3, Figure 9). When an abnormal case in the control group was determined as a case accompanied by rapid cognitive decline and eliminated, the specificity increased up to 0.889 (in parentheses of Figure 8-3).

Correlation of DO and severity of disease

Finally, the correlation of DO and severity of disease in view of clinical symptoms was examined (Figure 9). In AD (Figures 9A and B) and FTLD (Figures 9C and D), a negative correlation was observed between MMSE and DO (Figures 9A and C) or between frontal assessment battery (FAB) and DO (Figures 9B and D). This means that DO reflects seriousness of clinical symptoms in these dementia cases. However, such a correlation was not found in ALS cases between ALS functional evaluation scale-R (ALSFRS-R) and DO (Figure 9E). The reason for this is unknown; however, there is a possibility that more rapid progression of ALS than that of AD and FTLD may be involved. For example, in some cases, the actual rate of pathological progression does not reflect the severity of mobility impairment of ALS at a predetermined time point.

From the results of this Example, the followings were found.

It has been reported that phosphorylation of MARCKS at Ser46 occurs in neurites at an extremely early stage of AD via the HMGB1-TLR4 signal transduction pathway, which is induced by a damage warning molecule, HMGB1, released from adjacent neuron cells accompanied by intercellular accumulation of A$\beta$ (Fujita, K. et al., Sci. Rep. 6, 31895 (2016)). In this Example, MARCKS phosphorylated at Ser46 was evaluated based on the findings so far described and in expectation that it is used as a biomarker serving as an indicator for neurite degeneration and available in the early pathological stage and in the cerebrospinal fluids (CSFs) of three diseases, AD, FTLD, and ALS.

In all neurodegenerative diseases, the specificity of pSer46-MARCKS in the cerebrospinal fluid (CSF) was extremely high; however the sensitivity thereof was not so high (Figures 1-1 and 1-2). To overcome difficulties of pSer46-MARCKS as a biomarker, integration of the values of non-phosphorylated MARCKS and pSer46-MARCKS was made to produce a new parameter called DO (Figure 8-1). It was confirmed that DO is a good parameter having both a high sensitivity and a high specificity and sufficiently reflects severity of a disease in AD and FTLD. In order to optimize sensitivity and specificity in three neurodegenerative diseases, the ideal upper limit of normal DO was further investigated by using numerical simulation. As a result, if an "upper limit of normal DO" within the range of 50.26 to 54.24 was used, the sensitivity of AD, FTLD and ALS became 0.875, 0.857 and 1.00 respectively; and a specificity of 0.800 was obtained in all disease cases (Figure 8-1 to 8-3 and Figure 9).

Unexpectedly, DO commonly changed in a plurality of neurodegenerative diseases. Furthermore, DO and pSer46-MARCKS by itself were high in FTLD than in AD. The results indicate that DO and pSer46-MARCKS are not disease-specific markers but are indicators commonly seen for pathological processes of three neurodegenerative diseases. Previous studies revealed that pSer46-MARCKS abnormally increases even before histological formation of A$\beta$ aggregates (Tagawa, K. et al., Hum. Mol. Genet. 24, 540-58 (2015); Fujita, K. et al., Sci. Rep. 6, 31895 (2016)). Thus, DO and pSer46-MARCKS in the early stages of AD, FTLD, and ALS probably reflect neurite degeneration.

The biological significance of integration of non-phosphorylated MARCKS value to produce a more satisfactory biomarker, is not clear. However, both pSer46-MARCKS and non-phosphorylated MARCKS are released from decomposed neurites and active phosphorylation of MARCKS does not occur in the terminal stage of neurons. For the reason, the DO value obtained by integration with non-phosphorylated MARCKS is a marker more satisfactory than a marker using pSer-MARCKS alone.

[Example 2] Study on Parkinson's disease (PD) and dementia with Lewy bodies (DLB)

Methods

Mouse PD/DLB models

Normal humanized $\alpha$-Syn-BAC-Tg mice were produced in accordance with the method described in Yamakado et al (2012) Neurosci Res 73: 173-177.

More specifically, a BAC-Tg construct (PAC AF163864 and BAC AC097478, including a 28 kb 5'-flanking sequence and 50 kb 3'-flanking sequence in addition to all human genes) was micro-injected into C57BL6/J eggs to produce homozygous $\alpha$-Syn-Tg mice. GBA-hetero-KO mice were purchased from the Jackson Laboratory (B6.129S6-Gbatm1Nsb/J, stock number 003321) and mated with humanized $\alpha$-Syn-BAC-Tg mice. The obtained normal humanized α-Syn-BAC-Tg/GBA-hetero-KO (homo/hetero) mice were maintained as a lineage.

Mating of α-Syn-BAC-Tg/GBA-hetero-KO mice were repeated for 10 generations or more and the mice of 1, 6, and 24 months old (N = 3) were used for immunohistochemical and biological analyses.

Immunohistochemical analysis

For immunohistochemical analysis, a mouse or human brain was fixed with 4% paraformaldehyde and embedded in paraffin.

The brain sections embedded in paraffin were deparaffinized, rehydrated, activated in antigen (irradiated with microwave in a 10 mM citrate buffer, pH 6.0, at 100°C for 5 minutes. The irradiation process was repeated three times), and cooled to room temperature (RT). For staining of phospho-α-Syn, the sections were further activated in 98% formic acid (WAKO, 066-00461) at room temperature for 5 minutes.

The sections were washed twice with PBST (PBS containing 0.1% Tween-20) for 5 minutes, treated with PBS containing 0.5% Triton X-100 for 20 minutes and washed with PBST three times for 5 minutes. After blocked (with 10% FBS at 37°C for 30 minutes), the sections were continuously incubated in 2% FBS and 0.1% Triton X-100, together with a primary antibody (mouse anti-phospho-α-Syn (Ser129) (1:2000, WAKO, 015-25191)) at 37°C for 60 minutes; mouse anti-α-Syn (1:1000, Abcam plc., ab27766) at 4°C for 12 hours; rabbit anti-phospho-MARCKS (Ser46) (1:1000, GL Biochem (Shanghai) Ltd.) or a mouse anti-ubiquitin antibody (1:1000, Cell Signaling Technology, Inc., 3936S) at 37°C for 120 minutes; or rabbit anti-phospho-ERK1/2, mouse anti-MAP2 (1:100, santa cruz, sc-32791) or mouse anti-GFAP (1:2000, sigma, C9205) that detects mouse Thr203/Tyr205-ERK1/Thr183/Tyr185-ERK2 and human Thr202/Tyr204-ERK1/Thr185/Tyr187-ERK2 (1:250, Cell Signaling Technology, Inc., #4370) at 4°C for 12 hours, and using a secondary antibody (Alexa Fluor labeled anti-mouse IgG (1:1000, Invitrogen Corporation) or Cy3 labeled anti-rabbit IgG (1:500, Jackson ImmunoResearch Laboratories, Inc.) at RT for 60 minutes. A pSer46-MARCKS antibody was produced and the quality thereof was evaluated in accordance with the method described in Fujita et al., (2016) Sci Rep 6:31895.

For double labeling with pSer129-α-Syn and ubiquitin, an anti-pSer129-α-Syn antibody was labeled by use of Zenon Alexa Fluor 488 mouse IgG1 labeling kit (Z-25002, Invitrogen Corporation). The nuclei were stained with DAPI (Dojindo Laboratories, D523). Images were produced in accordance with confocal microscopy: Olympus FV1200 IX83 (Olympus Corporation).

Western blotting

Mouse cerebral cortex and human temporal lobe tissue were dissolved in an extraction buffer containing 2% SDS, 1 mM DTT, and 10 mM Tris-HCl (pH 7.5) and homogenized by Dounce glass homogenizer with 20 strokes on ice. The resultant crude extracts each were centrifuged at 16,000 g and 4°C for 10 minutes, separated by 1% SDS-PAGE with a same-volume sample buffer (0.1 M Tris-HCl pH 7.5, 4% SDS, 20% glycerol, and 12% β-mercaptoethanol), transferred to polyvinylidene difluoride membrane (Immobilon-P, Merck Millipore) in accordance with semi-dry method and blocked with 5% milk or 2% broth. Dissolution was carried out with BSA (% BSA) in TBST (10 mM Tris/HCl pH 8.0, 150 mM NaCl, and 0.05% Tween-20). A reaction was made with the following primary and secondary antibodies diluted with Can Get Signal solution (Toyobo Co., Ltd.). The primary and secondary antibodies were diluted as follows: mouse anti-phospho-α-Syn (Ser129) (1:5000, WAKO, 015-25191); mouse anti-α-Syn (1:5000, Abcam plc., ab27766) at 4°C for 12 hours; rabbit anti-phospho-MARCKS (Ser46) (1:200,000, GL Biochem (Shanghai) Ltd.) at 37°C for 120 minutes; a mouse anti-ubiquitin antibody (1:5000, Cell Signaling Technology, Inc., 3936S) at 4°C for 12 hours; rabbit anti-phospho-ERK1/2 (Thr203/Tyr205 (mouse)-ERK1/Thr183/Tyr185 (mouse)-ERK2, Thr202/Tyr204 (human)-ERK1/Thr185/Tyr187 (human)-ERK2) (1:10,000, Cell Signaling Technology, Inc., #4370); or rabbit anti-ERK1/2 (1:5,000, Cell Signaling Technology, Inc., #4695S); an anti-mouse IgG HRP conjugate (1:3000, GE Healthcare, NA931VA) and anti-rabbit IgG (1:3000, GE Healthcare, NA934VS). To detect bands using LAS4000 (GE Healthcare), ECL prime (GE healthcare, RPN2232) or SCL select (GE healthcare, RPN2235) was used.

Immunoprecipitation

Mouse and human brain specimens each were dissolved in TNE buffer (10 mM Tris-HCl (pH 7.5), 10 mM NaCl, 1 mM EDTA, 1% NP-40, 0.5% protease inhibitor cocktail, 0.5% phosphatase inhibitor cocktail). An aliquot was taken, incubated with a 50% slurry of protein G sepharose beads (GE healthcare) and subsequently centrifuged (2000 × g) for 3 minutes. The supernatant was incubated with 1 μg of a rabbit anti-pSer46-MARCKS antibody or a rabbit anti-pSer129-τ-synuclein antibody at 4°C overnight. Incubation was made with protein G sepharose beads (GE healthcare) for 4 hours. Washing was made with TNE buffer and elution was made with the sample buffer.

Collection of peripheral blood cells from AD model mouse

Peripheral blood cells (polymorphonuclear leukocytes (PMNs) and mononuclear cells (MCs)) were collected by using Polymorphprep solution (Alere Technologies AS) in accordance with the manufacturer's protocol. In short, 1 mL of venous blood containing EDTA (final concentration: 2.0 mM) was carefully layered on 1 mL of Polymorphprep contained in a 15 mL tube, centrifuged at room temperature and 500 × g for 30 minutes, and thereafter, the plasma was removed to obtain a layer containing PMN and MC. An aliquot was taken, diluted with 0.45% NaCl and centrifuged at 400 × g for 10 minutes to obtain a cell pellet. The pellet was dissolved with a lysis buffer (10 mM Tris-HCl (pH 7.5), 0.2% SDS, 0.5% protease inhibitor cocktail, and 0.5% phosphatase inhibitor cocktail), added to a mixture of 62.5 mM Tris-HCl, pH 6.8, 2 SDS, 2.5% (v/v) 2-mercaptoethanol, 5% (v/v) glycerol, and 0.0025% (w/v) bromophenol blue, and subjected to SDS-PAGE.

Mass spectrometry

For phosphoprotein analysis, a phosphorylated peptide was prepared in accordance with the method described in Fujita et al., (2016) Sci Rep 6:31895 and Tagawa et al., (2015) Hum Mol Genet 24:540-558.
More specifically, brain extracts derived from a mouse and human cerebral cortex were denatured with a surfactant and heat treatment, and then, cysteine residues were reduced and blocked with alkylation. The protein samples were digested with trypsin.
Labeling was made with iTRAQ Reagent multiplex kit (SCIEX Ins.). Fraction was made by powerful cation exchange chromatography and the amount of a phosphopeptide was increased by use of Titansphere Phos-TiO kit (GL Sciences Inc.). Individual fractions were analyzed by LC-MS/MS system (Triple TOF 5600 system, Eksigent LC system, SCIEX Ins.) and C18 column (0.1 × 100 mm; KYA Technologies Corporation). The ion spray voltage was 2.3 kV and information dependent acquisition (IDA) was set at 400 to 1250 m/z.

Data analysis

Mass spectrum data of a peptide were acquired and analyzed by Analyst TF (version 1.6) (AB SCIEXA). The protein corresponding to the result was searched for in UniProtKB/Swiss-Prot (downloaded on June 22, 2010 from http://www.uniprot.org) and Paragon algorithm was employed (ProteinPilot (version 4) (AB SCIEX) (Shilov et al. (2007) Mol421 Cell Proteomics 6:1638-1655)).
Allowable tolerance in the peptide search by ProteinPilot was set at 0.05 Da for MS and 0.10 Da for MS/MS analysis. If a long protein was identified, it was excluded by use of Pro Group algorithm (AB SCIEX).
The confidence score for identification of a protein or peptide was calculated by ProteinPilot and used as a confidence threshold. The threshold for detection was set at the degree of confidence of 95%. The peptide having a confidence of >95% was accepted as an identified peptide.
Proteins were quantified through analysis of iTRAQ reporter group in MS/MS spectra produced during fragmentation by a mass spectrometer. In quantification of peptides and proteins, under the assumption that the total amount of signals derived from individual iTRAQ is equal, bias correction was employed in order to normalize signals between different iTRAQ reporters. In quantification of a peptide, a bias correction option was used for normalizing different iTRAQ signals.
The peptide ratio was calculated as the ratio of the reporter signal in a disease model relative to that of a control model after bias correction. The details of the preparation are described in the manual of AB SCIEX.
The results of peptide summary from ProteinPilot were outputted as an Excel file for further data analysis. The amount of a peptide fragment was calculated as a signal-intensity geometric mean of a plurality of MS/MS fragments containing phosphorylation sites.
Biological difference between all disease groups and the control group was evaluated by the Welch test. For correction of multiple tests, p value was controlled by use of the Benjamini-Hochberg procedure.
Phosphopeptides changed in a plurality of disease groups were identified and selected for further analysis.
Human brain samples of the temporal pole and the occipital pole used for human-brain proteome analysis were obtained by dissection from 5 AD patients, 5 DLB patients, and 5 age-matched normal control patients and deeply frozen (-80°C) within 1 hour after death.

Results

Increase of pSer46-MARCKS level in human AD and DLB phosphorylated proteome

Following previous analysis of brain samples from AD mouse models and human AD patients (Tagawa et al.,

(2015) Hum Mol Genet 24:540-558), comprehensive analysis for phosphorylated proteins was carried out using dead human DLB patients. Tips of the occipital region and temporal region of the brains of pathologically pure AD (5 males) and DLB (5 females) patients were used.

In this case, the pathologically pure dead brain was selected. Intracellular $\alpha$-Syn aggregates were observed within a neuron but an extracellular A$\beta$ aggregate or cytoplasmic tau/TDP43 lesion was not observed. From the comparison, it was found that a phosphorylation site is shared between the AD sample and DLB sample.

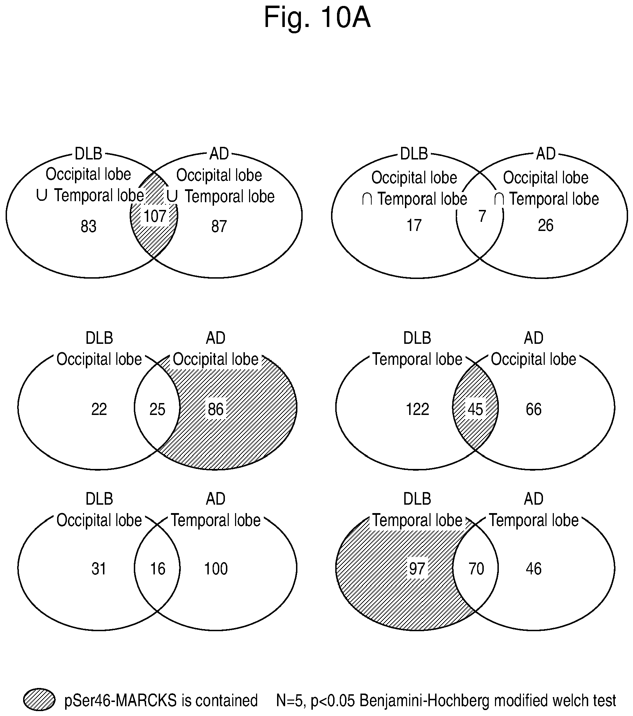

pSer46-MARCKS modified by phosphorylation increased in level in an AD mouse model and the dead human AD brain (Fujita et al., (2016) Sci Rep 6:31895) (Figure 10A).

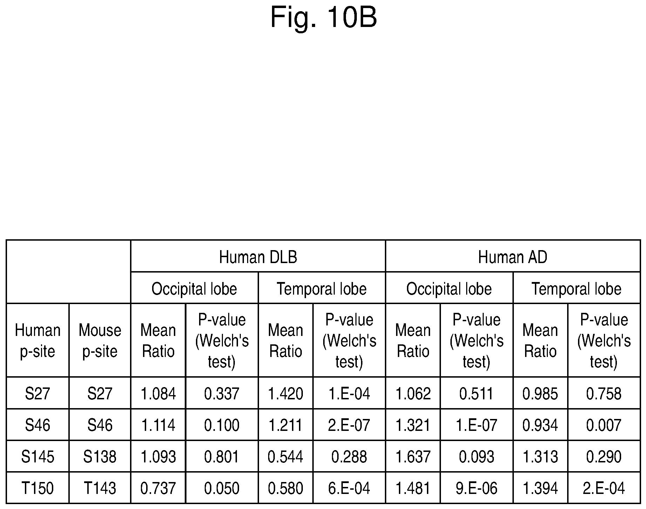

Interestingly, the pSer46-MARCKS level increased not in the occipital lobe but in the temporal lobe of DLB; however, the opposite pattern was seen in AD (Figure 10B) (more specifically, increase was seen in the occipital lobe and not in the temporal lobe). In 5 × FAD mice, the pSer46-MARCKS level increased in the early stage (preclinical/pre-aggregation stage) of a disease but returned to normal in the late stage thereof (Fujita et al., (2016) Sci Rep 6:31895).

Accordingly, there is a possibility that the difference in pSer46-MARCKS level between the two lobes reflects the lobe predominantly involved in neurodegeneration in each disease. The results obtained by mass spectrometry suggested that a common change occurred in AD and PD/DLB human brains, and investigation on whether pSer46-MARCKS reflects preclinical/pre-aggregation stage of PD/DLB or not is required.

Increase of pSer46-MARCKS in human DLB immunohistochemically analyzed

Whether the level of pSer46-MARCKS was increased or not was examined in the dead human DLB brain. For this purpose, the present inventors stained a sample of the occipital lobe of a DLB patient, and found that the dendrite of the apex marked with MAP2 was co-stained with pSer46-MARCKS (Figure 11A).

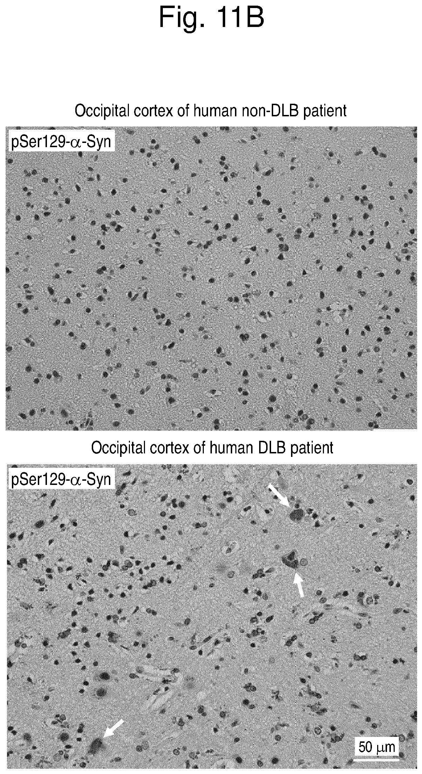

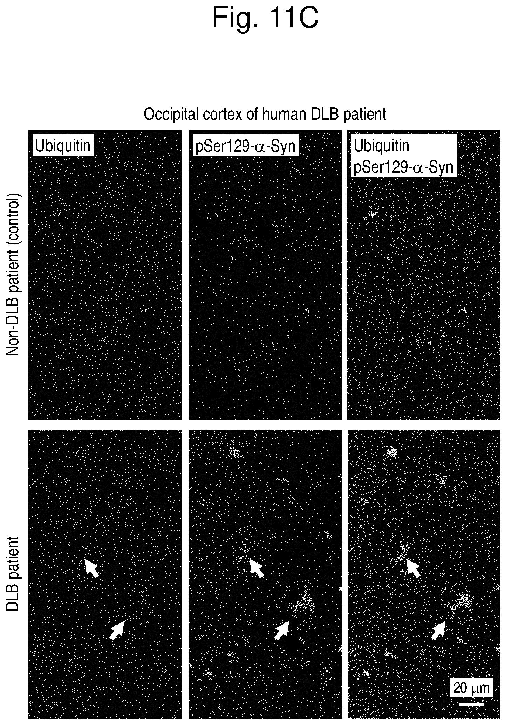

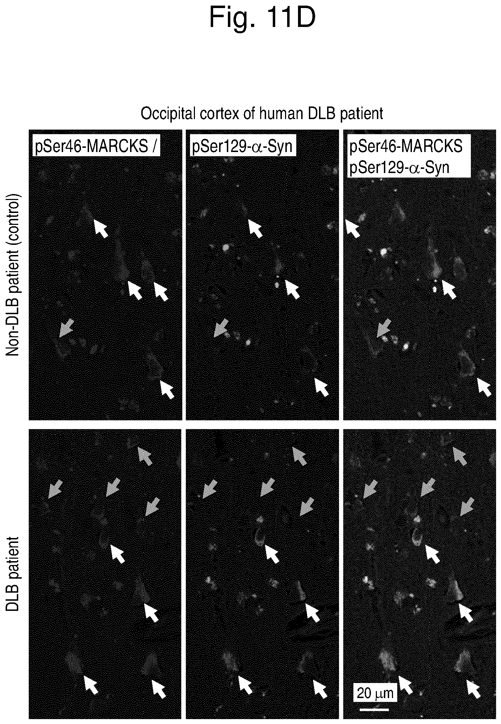

It was also confirmed that pSer129-$\alpha$-Syn cytoplasmic inclusion bodies (Figure 11B) mostly ubiquitinated are contained in this brain region (Figure 11C). Since something that looked like aggregates was observed in cytoplasm staining of pSer46-MARCKS (Figure 11A), whether or not these aggregate-like materials were $\alpha$-Syn aggregates was examined. It was found that a half of the pSer46-MARCKS-positive structures in the neuron cytoplasm was stained with pSer129-$\alpha$-Syn (yellow arrow). However, the other half was not strongly stained with pSer129-$\alpha$-Syn (Figure 11D, red arrow).

pSer46-MARCKS-positive/pSer129-$\alpha$-Syn-negative cells can correspond to peripheral neurons, which are indirectly influenced by HMGB1 released from damaged neuron at which pSer129-$\alpha$-Syn were accumulated, suggesting that pathological state is continuously developed in the human brain even in the DLB terminal stage.

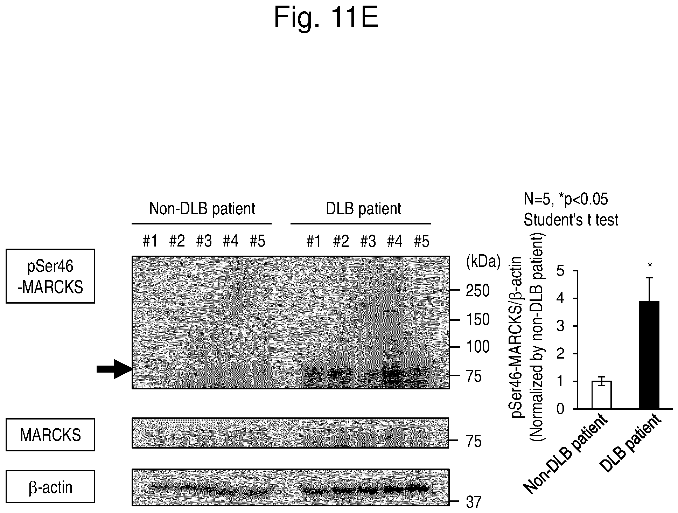

To confirm the above results of the immunohistochemical analysis, the present inventors conducted western blotting of pSer46-MARCKS and total MARCKS in the occipital lobes of a dead human DLB patient and a non-DLB control patient (Figure 11E). The pSer46-298 MARCKS level increased in the DLB patient.

Increase of pSer46-MARCKS in humanized $\alpha$-Syn-Tg mouse

To determine the point from which pSer46-MARCKS started increasing in the brain, 1-, 6-, and 24-month-old humanized $\alpha$-Syn-BAC-Tg/GBA-hetero-KO mice, which did not show motor dysfunction or abnormal behavior, were analyzed.

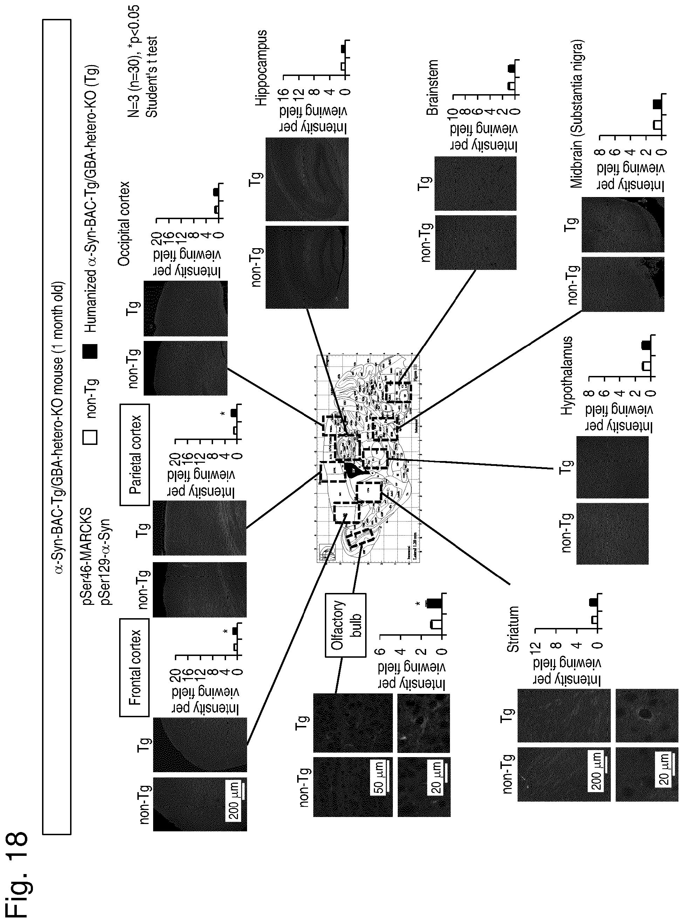

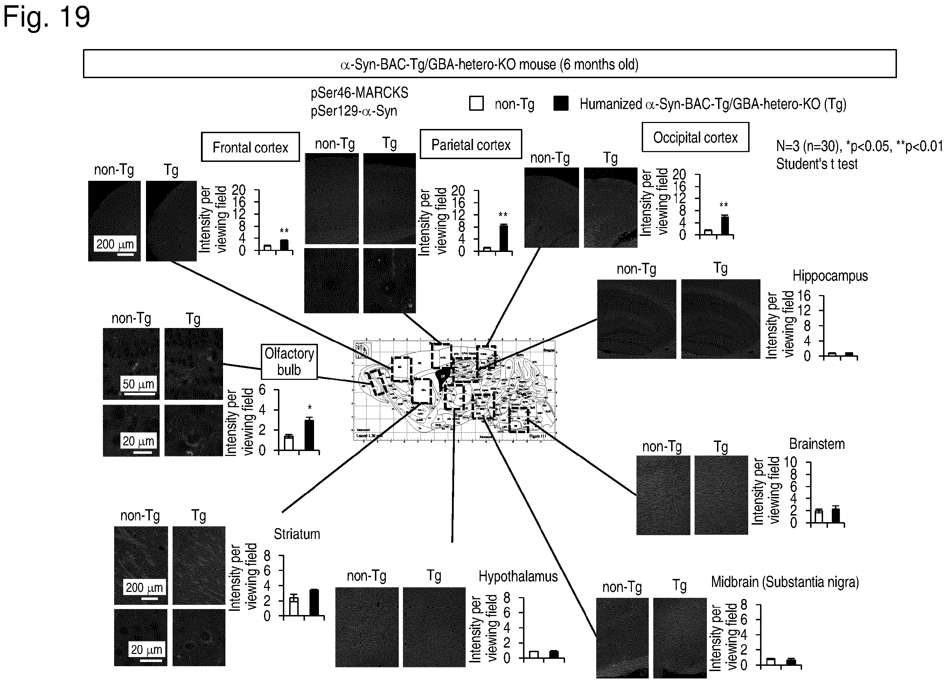

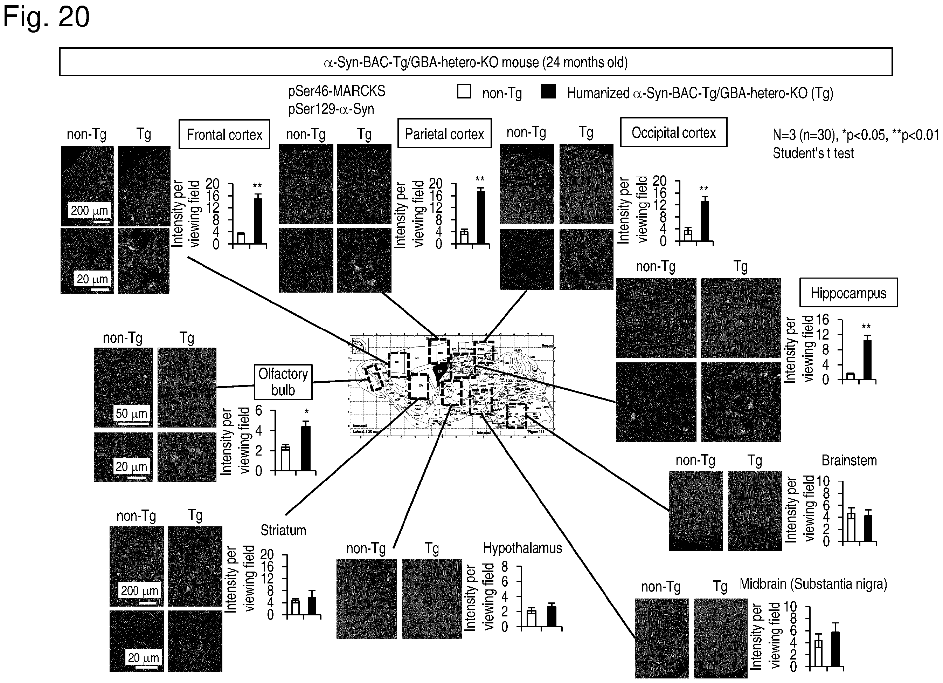

In the mice, pSer46-MARCKS signals in the olfactory bulb, frontal cortex, and parietal cortex slightly increased in a month (Figure 12, Figure 18). Thereafter, the positively stained area in the case of pSer46-MARCKS expanded in 6 months towards the parietal cortex and the occipital cortex (Figure 12, Figure 19) and did not increase in the hippocampus before 24 months (Figure 12, Figure 20).

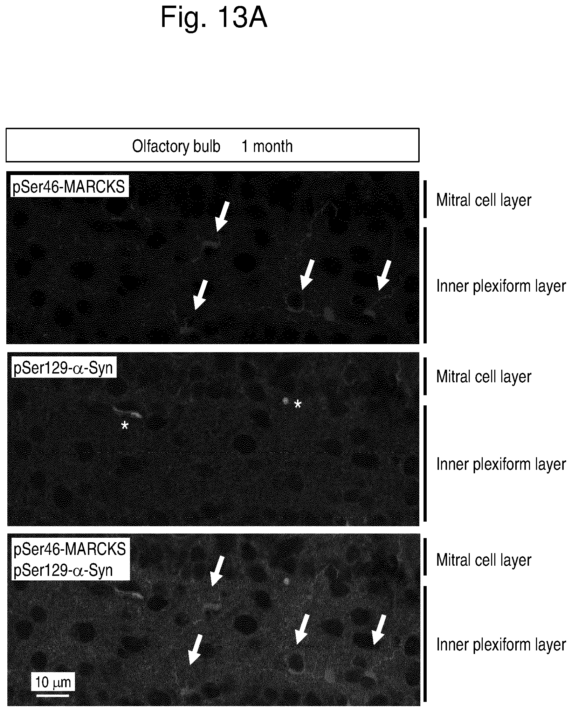

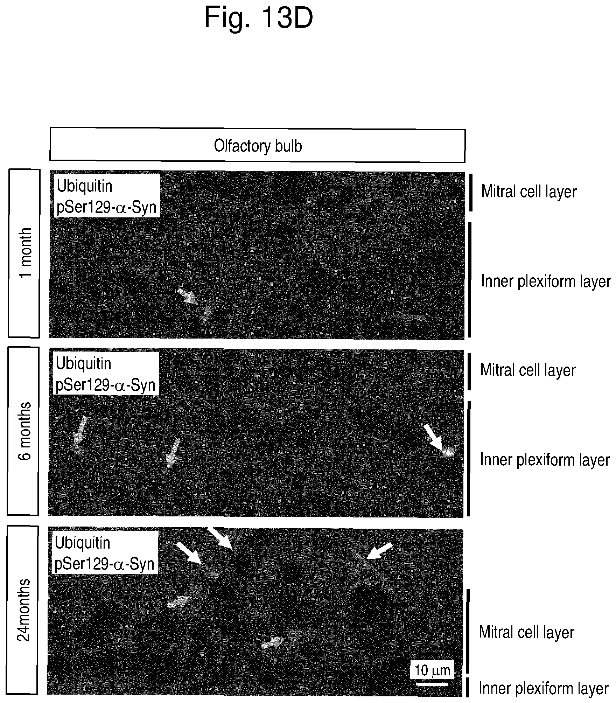

In the neurons of the endoderm layer and the mitral cell layer of the olfactory bulb up to 24 months, definite cytoplasmic $\alpha$-Syn aggregates stained with an anti-phosphorylated $\alpha$-Syn (pSer129-$\alpha$-Syn) antibody were not detected (Figures 13A to C). Figures 13A to C show high magnification images of the olfactory bulb of a humanized $\alpha$-Syn-BAC-Tg/GBA-hetero-KO mouse co-stained with antibodies to pSer46-MARCKS and pSer129-$\alpha$-Syn. Dot-like staining of pSer46-MARCKS (white arrow) and pSer129-$\alpha$-Syn (asterisk) in the cytoplasm was detected in the mouse of 1 month old; however, aggregates of pSer129-$\alpha$-Syn in the cytoplasm were only detected in the mouse of 24 months old. The presence of $\alpha$-Syn-skein- or Lewy neurite-like structure was observed in these layers in the mouse of 1 month old and the number of the structures increased during the aging process. These structures were not simultaneously stained with an anti-ubiquitin antibody in the mouse of 1 month old but apparently co-stained in the mouse of 6 months old or more (Figure 13D).

Interestingly, the number of pSer46-MARCKS-positive neurites was larger than the number of p-$\alpha$-Syn-co-stained neurites.

To sum up, these results showed that a neurite change detected by pSer46-MARCKS in at least the PD/DLB mouse model precedes formation of α-Syn ubiquitin-positive aggregate (Table 1).

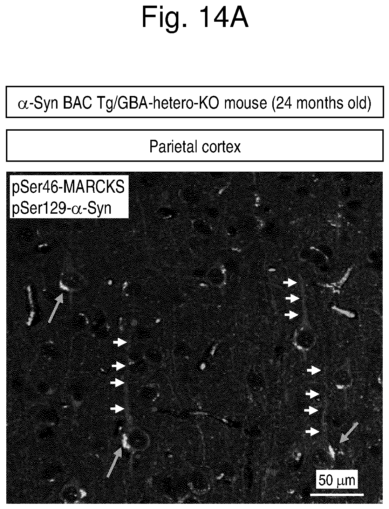

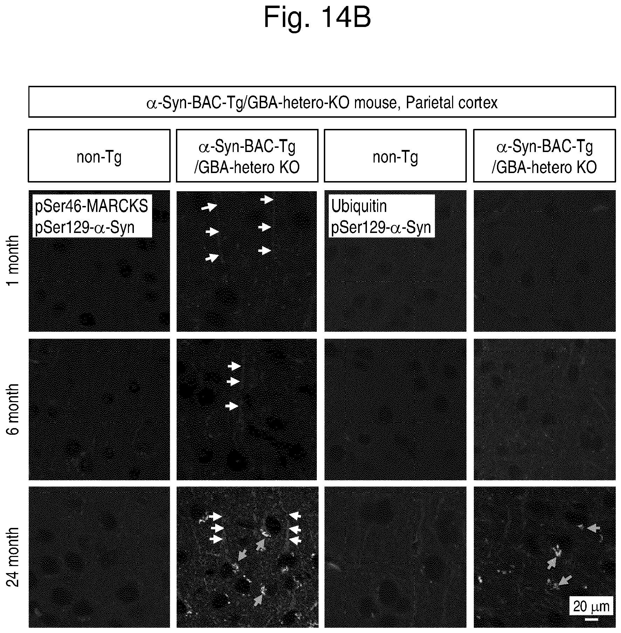

In the mouse of 24 months old, cytoplasmic α-Syn aggregates were detected in the parietal cortex (Figure 14A). In aged cortical neurons, relationship between pMARCKS and p-α-Syn and the relationship between p-α-Syn and ubiquitin were similar to those in the olfactory neurons (Figure 14B).

To confirm these results, the present inventors carried out western blot analysis of pSer46-MARCKS, pSer129-α-Syn, and ubiquitin of the cerebral cortex tissues of 1-, 6-, and 24-month-old mice (Figure 15). The level of phosphorylated MARCKS increased in the 1-, 6-, and 24-month-old mice. 6 months later, α-Syn and ubiquitin phosphorylated were observed and the level thereof sufficiently increased (Figure 15).

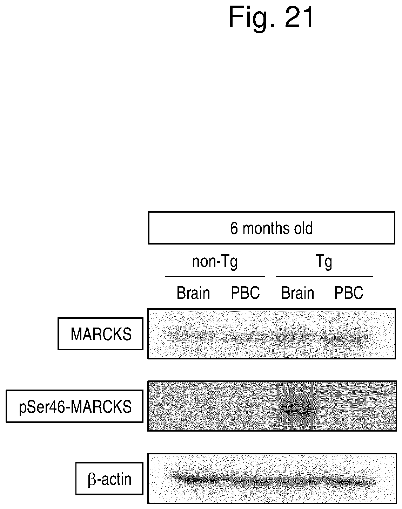

The present inventors further examined the level of pSer46-MARCKS in the peripheral blood cells and found that the level thereof is extremely lower than that in the brain (Figure 21).

Interaction between MARKCS and α-Syn in DLB brain

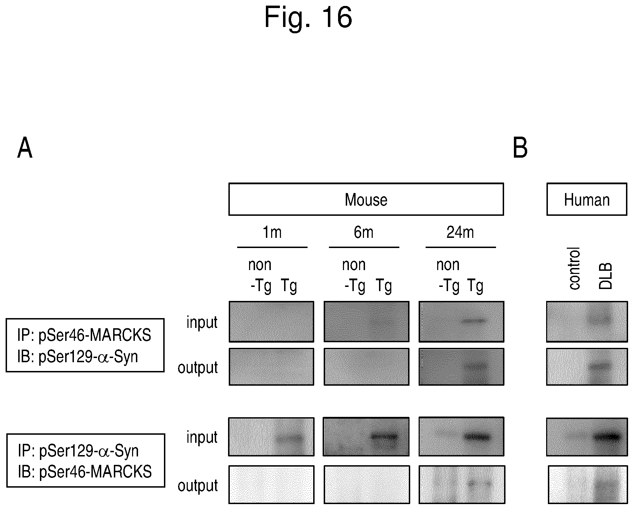

In consideration that the half of the cytoplasm aggregates is double-positive to pSer46-MARCKS and pSer129-α-Syn (Figure 11D), the present inventors examined biochemical interactions between these two phosphoproteins. Anti-pSer46-MARCKS and pSer129-α-Syn derived from the brain sample (whole cerebral cortex) (Figure 16A) of 24-month-old α-Syn-BAC-Tg/GBA-hetero-KO mouse (Figure 16A) and the human DLB patient (Figure 16B) were subjected to simultaneous immunoprecipitation. Reverse precipitation was carried out with the anti-pSer129-α-Syn antibody. As a result, pSer46-MARCKS was co-precipitated in the mouse model and the human patient (Figures 16A and B). The result indicates that these two phosphoproteins biochemically interact.

Activation of upstream kinase in the DLB brain

Finally, upstream kinase, which phosphorylates MARCKS at Ser46, was examined. MARCKS is a typical substrate of PKC and known as myristoylated alanine rich C kinase substrate. PKAα was reported that Ser159, Ser163, and Ser170 are phosphorylated. On the other hand, it has been elucidated that Erk1/Erk2 (= MAPK3/MAPK1) phosphorylates MARCKS at Ser46 in place of PKC. Similarly, another group also reported that MAPK phosphorylates MARCKS in the hippocampal neurons. The accurate sites to be phosphorylated by MAPK were determined by their studies.

Accordingly, using a rabbit monoclonal antibody, which detects Erk1 phosphorylation at Thr202/Tyr204 and Erk2 phosphorylation at Thr185/Tyr187, immunohistochemical analysis and western blot were carried out to examine activation of Erk1/2 (Figure 17A).

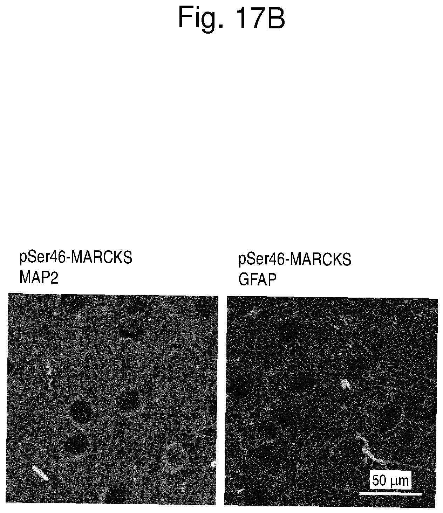

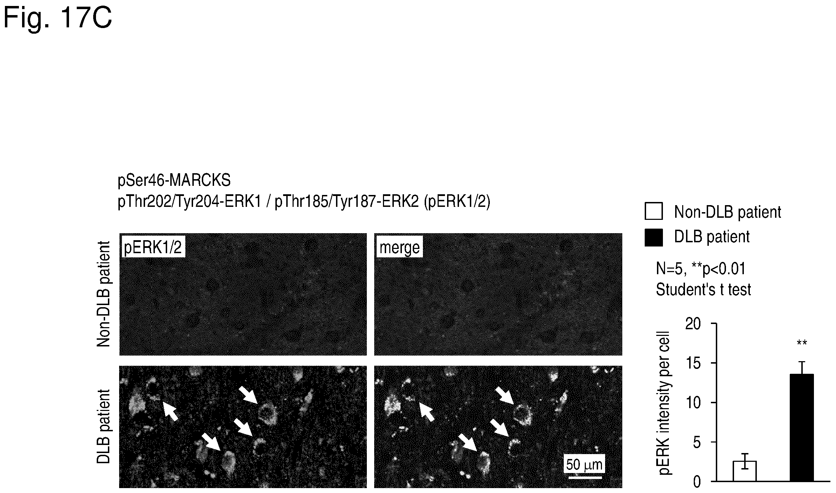

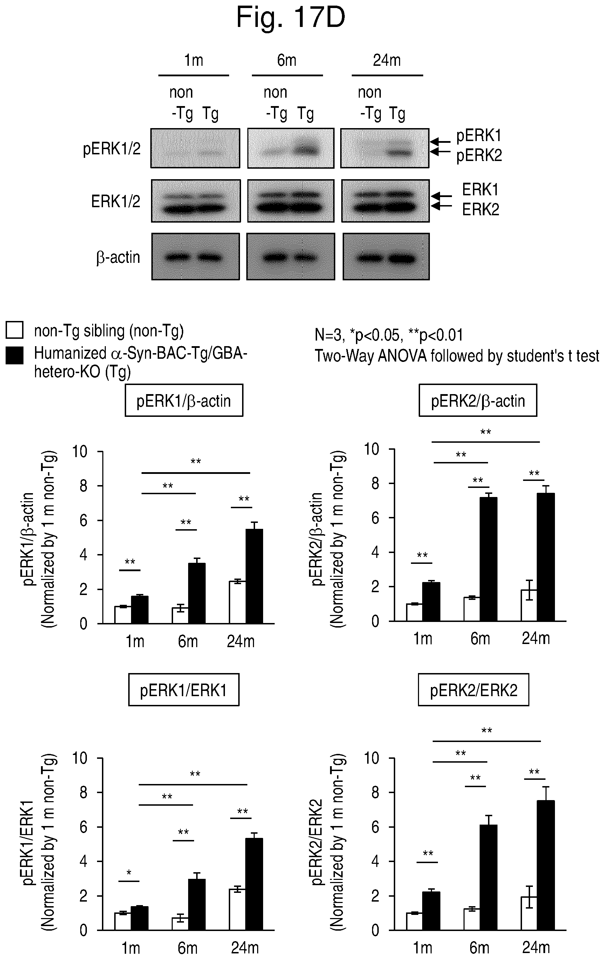

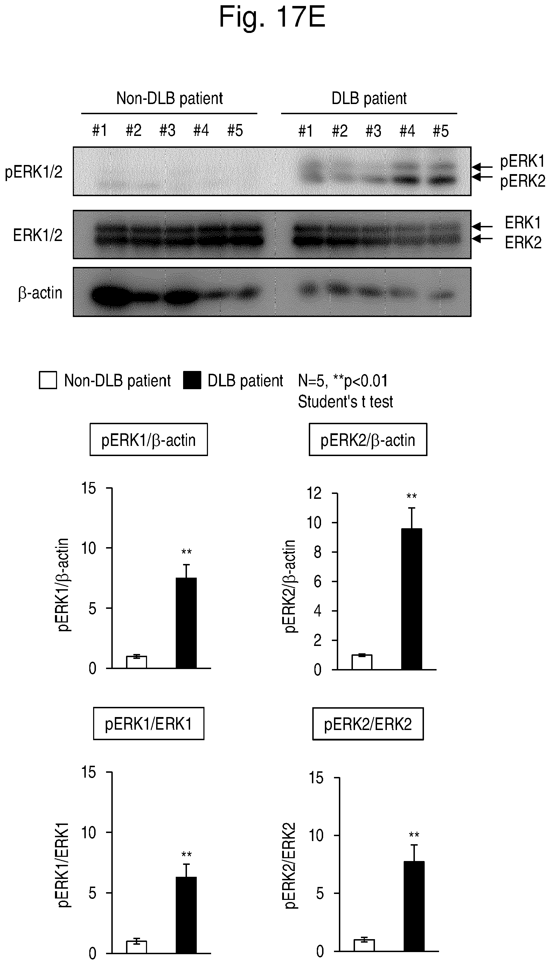

As expected, immunostaining of pErk1/2 increased in the cerebral cortex of α-Syn-BAC-Tg/GBA-hetero-KO mouse (Figure 17A). Cells were simultaneously stained with MAP2 but not co-stained with GFAP and found to be neurons (Figure 17B). Staining of pErk1/2 was consistent with the relationship between these enzyme-substrates and co-present with pSer46-MARCKS in the same neuron (Figure 8A). In the occipital cortex of a DLB patient, the same co-staining indicating an increase of pSer46-MARCKS and pErk1/2 was observed (Figure 17C). It was further confirmed by western blot analysis that pErk1/2 increased in the cerebral cortex of α-Syn-BAC-Tg/GBA-hetero-KO mouse (Figure 17D) and the DLB patient (Figure 17E). These analyses in combination demonstrate an abnormal increase of Erk1/2 phosphorylation and age-dependent enhancement thereof in the cortical neurons under PD/DLB pathology (Figures 17A to E).

Based on the results of the above Example, the followings were found:

The feature of neurite degeneration in the pre-aggregation stage of AD pathology, that is, the level of MARCKS phosphorylated at pSer46, increases in PD/DLB medical conditions of both mouse models and human patients.

The immunohistochemical staining pattern of pSer46-MARCKS was analogous between AD and PD/DLB lesions. In mouse models, the neurite degeneration found by pSer46-MARCKS was initially detected in the olfactory bulb, and thereafter, significantly found in the occipital region and temporal cortex (Table 1). Table 1 collectively shows staining patterns of pSer46-MARCKS, pSer129-α-syn, and ubiquitin in humanized α-Syn-BAC-Tg/GBA-hetero-KO mouse. The reference symbol "+/-" indicates that the ratio of positive staining of cells was less than 10% in a background mouse, in other words, staining was not observed. The reference symbol "+" indicates that positive staining was observed in 10 to 50% of cells of Tg mouse. The reference symbol "++" indicates that positive staining was observed in 50% or more of cells of Tg mouse.

The pattern matches with the progression of neurodegeneration as suggested in human PD/DLB pathology, that is, the earliest lesion appears in the olfactory bulb and finally appears predominantly in the occipital lobe.

[Table 1]

| Summary of pathological changes in α-Syn-BAC-Tg/GBA-hetero-KO mouse | | | | | | | | | | | | | | | | | | | | |
|---|---|---|---|---|---|---|---|---|---|---|---|---|---|---|---|---|---|---|---|---|
| α-Syn- Tg/ GBA-hetero-KO mouse | | 1 month | | | | | | 6 months | | | | | | 24 months | | | | | | |
| Region | | pMARCKS (Ser46) | | pα-Syn | | Ubiquitin | | pMARCKS (Ser46) | | pα-Syn | | Ubiquitin | | pMARCKS (Ser46) | | pα-Syn | | Ubiquitin | | |
| | | non -Tg | Tg | non -Tg | Tg | non -Tg | Tg | non -Tg | Tg | non -Tg | Tg | non -Tg | Tg | non -Tg | Tg | non -Tg | Tg | non -Tg | Tg | |
| Cortex | Frontal cortex | - | +/- | - | - | - | - | - | + | - | +/- | - | - | - | ++ | - | + | - | + | |
| | Temporal cortex | - | +/- | - | - | - | - | - | + | - | +/- | - | - | - | ++ | - | + | - | + | |
| | Occipital cortex | - | +/- | - | - | - | - | - | + | - | +/- | - | - | - | ++ | - | + | - | + | |
| Hippocampus | | - | - | - | - | - | - | - | - | - | - | - | - | - | ++ | - | + | - | + | |
| Hypothalamus | | - | - | - | - | - | - | - | - | - | - | - | - | - | - | - | - | - | - | |
| Striatum | | - | +/- | - | - | - | - | - | +/- | - | - | - | - | - | +/- | - | - | - | - | |
| Midbrain (substantia nigra) | | - | - | - | - | - | - | - | - | - | - | - | - | - | - | - | - | - | - | |
| Cerebellum | | - | - | - | - | - | - | - | - | - | - | - | - | - | - | - | - | - | - | |
| Brainstem | | - | - | - | - | - | - | - | - | - | - | - | - | - | - | - | - | - | - | |
| Olfactory bulb | Inner plexiform layer | - | + | - | +/- | - | - | - | + | - | + | - | + | - | ++ | - | ++ | - | + | |
| | Mitral cell layer | - | + | - | +/- | - | - | - | + | - | + | - | + | - | ++ | - | ++ | - | + | |

The second important conclusion in this study is that neurite degeneration histologically precedes disease-related protein aggregation. In a mouse model, an increase of pSer46-MARCKS precedes formation of ubiquitinated $\alpha$-Syn aggregates (Table 1).

The phenomenon taking place in chronological order in PD/DLB mouse model where pSer46-MARCKS precedes histological formation of aggregates was analogous to previous observation (extracellular staining of A$\beta$ aggregate was clearly observed) in AD model mouse, that is, pSer46-MARCKS was detected in the cytoplasm of neuron and neurite.

Using the model, the present inventors confirmed importance of pSer46-MARCKS at a biochemical level. These results strongly suggest that intracellular misfolded $\alpha$-Syn in the form of a monomer or an oligomer plays an important role in initiation of neurite degeneration rather than aggregates.

Interestingly, in a dead human brain, the level of pSer46-MARCKS increased not in the brain region seriously influenced (occipital lobe in DLB and temporal lobe in AD) but in the brain region not so seriously influenced by a disease (temporal lobe in DLB and occipital lobe in AD).

The reason of this phenomenon is still unknown. One of the possible reasons is that neurites in a seriously affected brain region are metabolically "burned out and cannot maintain a high level of pSer46-MARCKS."

Based on these observations, it is worth developing pSer46-MARCKS as a biomarker that can detect molecular pathology of PD/DLB in a super-early (pre-aggregation/preclinical) stage.

Further, it is useful to develop an assay based on mass spectrometry, assay based on ELISA, and/or PET. As described, if a high sensitive assay system is available, the system seems to directly lead to detection of super-early-stage pathological conditions in people having a risk of a neurodegenerative disease.

The present inventors preliminary examined whether pSer46-MARCKS is expressed in the peripheral blood cells (PBCs) such as red blood cells, granule cells, or lymphocytes, by western blot. If pSer46-MARCKS is expressed, it would be difficult to distinguish pSer46-MARCKS derived from PBC from that derived from the brain. In order to use pSer46-MARCKS as a biomarker for extracranial diagnosis, some tricks must be added. Fortunately, pSer46-MARCKS was not expressed in PBC at a detectable level corresponding to that in brain tissues of 5 x FAD mice (Figure 21). Possibility of pSer46-MARCKS as a biomarker in blood is supported.

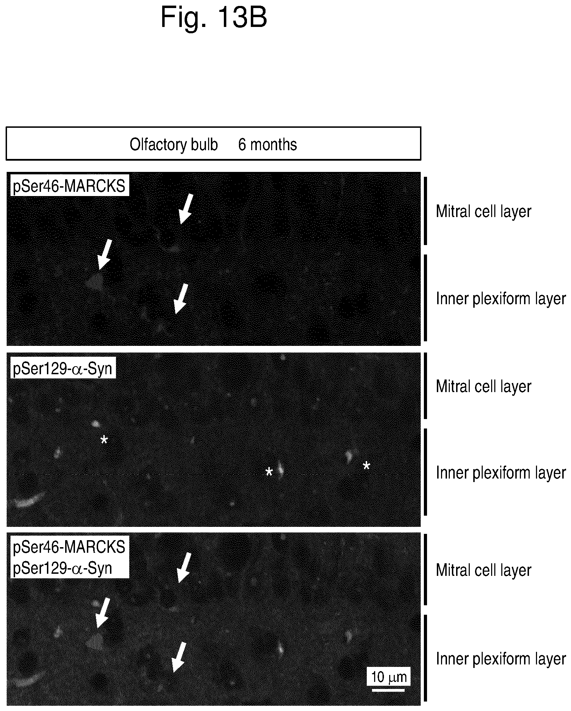

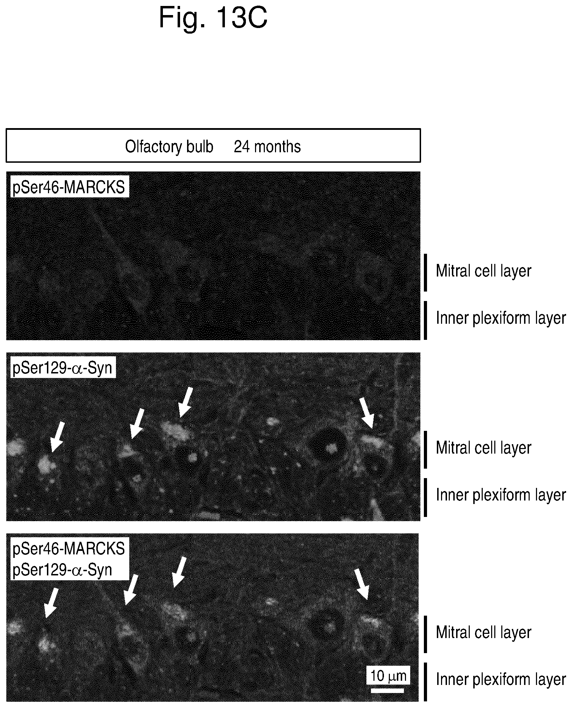

The present inventors found that pSer46-MARCKS and pSer129-$\alpha$-Syn cytoplasm aggregates are locally co-present (Figure 13, Figure 14A, Figure 14B) and that they biochemically interact (Figure 16). Interestingly, not only $\alpha$-Syn but also MARCKS are seemed to be essentially deregulated protein (IDP). In previous studies, it was found that $\alpha$-Syn is naturally denatured.

On the other hand, this idea was supported by the structural biological experiment previously reported, in which MARCKS was predicted as IDP by bioinformatics analysis using available algorithm (RONN v3.2, https://www.strubi.ox.ac.uk/RONN and IUPred, http://iupred.enzim.hu). There is a possibility that similar degenerative tendencies of $\alpha$-Syn and MARCKS serve as the base of their biochemical interactions. Furthermore, localization of both $\alpha$-Syn and MARCKS in dystrophic neurites attracts attention and is important in axon terminal function such as axon growth and actin network regulation.

Since a pathological cycle from neurite degeneration to cell death is repeated in the brain having a neurodegenerative disease until all neurons of the brain die, increases of pSer46-MARCKS and DO can be used as quantitative biomarkers for *in vivo* activity of neurodegeneration similarly as general quantitative indicators such as GOT and GPT for hepatocyte damage, though not disease-specific, in multiple liver disease and similarly to symptomatic patients and probably non-syndromic subjects. Examples of the invention provide a novel biomarker reflecting neurite degeneration of neurodegenerative diseases.

Industrial Applicability

The method of the present invention can be used for detecting a neurodegenerative disease selected from the group consisting of human Alzheimer's disease (AD), frontotemporal lobar degeneration (FTLD), amyotrophic lateral sclerosis (ALS), Parkinson's disease (PD), and dementia with Lewy bodies (DLB).

All publications, patents, and patent applications cited in the specification are incorporated herein by reference in their entirety.

**Claims**

1. A method for detecting a neurodegenerative disease selected from the group consisting of human Alzheimer's disease (AD), frontotemporal lobar degeneration (FTLD), and amyotrophic lateral sclerosis (ALS), comprising the steps of:

   (i) measuring a MARCKS protein phosphorylated at position 46 and a non-phosphorylated MARCKS protein in a test specimen collected from a subject;
(ii) calculating a DO value expressed by the following formula from the measured values obtained in (i):

[Formula 1]

$$DO = \sqrt{(pSer46\text{-}MARCKS)^2 + (non\text{-}phosphorylated\text{-}MARCKS)^2}$$

where "pSer46-MARCKS" represents the amount of a MARCKS protein phosphorylated at position 46; and "non-phosphorylated-MARCKS" represents the amount of a non-phosphorylated MARCKS protein; and
(iii) detecting a neurodegenerative disease based on the DO value as an indicator.

2. The method according to Claim 1, wherein the test specimen is cerebrospinal fluid.

3. The method according to Claim 1 or 2, wherein "pSer46-MARCKS" and "non-phosphorylated-MARCKS" are measured by mass spectrometry.

4. The method according to any one of Claims 1 to 3, wherein "pSer46-MARCKS" and "non-phosphorylated-MARCKS" in the formula are normalized values by the total amount of proteins in the test specimen.

5. The method according to any one of Claims 1 to 4, wherein, in step (iii), the DO value of the subject calculated is compared to a cutoff value previously specified and if the DO value is higher than the cutoff value, it is determined that a neurodegenerative disease is detected.

6. The method according to Claim 5, wherein the cutoff value of the DO value is 50 to 55.

7. A method for detecting a neurodegenerative disease selected from the group consisting of human frontotemporal lobar degeneration (FTLD), amyotrophic lateral sclerosis (ALS), Parkinson's disease (PD), and dementia with Lewy bodies (DLB), comprising the steps of:

(i) measuring a MARCKS protein phosphorylated at position 46 in a test specimen collected from a subject;
(ii) comparing the amount of the MARCKS protein phosphorylated at position 46 in the test specimen collected from the subject to the amount of the MARCKS protein phosphorylated at position 46 in a test specimen collected from a healthy person; and
(iii) determining that a neurodegenerative disease is detected in the subject if the amount of the MARCKS protein phosphorylated at position 46 in the test specimen collected from the subject is larger than the amount of the MARCKS protein phosphorylated at position 46 in the test specimen collected from the healthy person.

8. The method according to Claim 7, wherein the test specimen is cerebrospinal fluid.

9. The method according to Claim 7 or 8, wherein the MARCKS protein phosphorylated at position 46 is measured by mass spectrometry.

10. The method according to any one of Claims 7 to 9, wherein the amount of the MARCKS protein phosphorylated at position 46 is a value obtained by normalizing the measured value of the MARCKS protein phosphorylated at position 46 by the total amount of proteins in the test specimen or the total amount of MARCKS proteins in the test specimen.

11. A method for detecting a neurodegenerative disease selected from the group consisting of Parkinson's disease (PD) and dementia with Lewy bodies (DLB), comprising the steps of:

(i) measuring a MARCKS protein phosphorylated at position 46 in test specimens collected from the occipital lobe and temporal lobe of a subject;
(ii) comparing the amounts of the MARCKS protein phosphorylated at position 46 in the test specimens collected from the occipital lobe and temporal lobe of the subject to the amounts of the MARCKS protein phosphorylated at position 46 in test specimens collected from the occipital lobe and temporal lobe of a healthy person; and
(iii) determining that a neurodegenerative disease is detected in the subject if the amount of the MARCKS protein phosphorylated at position 46 in the test specimen collected from the temporal lobe of the subject is larger than the amount of the MARCKS protein phosphorylated at position 46 in the test specimen collected from the temporal lobe of the healthy person.

12. The method according to Claim 11, wherein the test specimen is cerebrospinal fluid.

13. The method according to Claim 11 or 12, wherein the MARCKS protein phosphorylated at position 46 is measured by mass spectrometry.

14. The method according to any one of Claims 11 to 13, wherein the amount of the MARCKS protein phosphorylated at position 46 is a value obtained by normalizing the measured value of the MARCKS protein phosphorylated at position 46 by the total amount of proteins in the test specimen or the total amount of MARCKS proteins in the test specimen.

15. The method according to Claim 11, wherein the MARCKS protein phosphorylated at position 46 is measured by positron emission tomography (PET) using a PET tracer for imaging the MARCKS protein phosphorylated at position 46.

16. The method according to Claim 15, wherein the PET tracer for imaging the MARCKS protein phosphorylated at position 46 is an antibody PET tracer prepared by labeling an antibody to the MARCKS protein phosphorylated at position 46 with a positron nuclide.

## Fig. 1-1

A

● pSer46-MARCKS
(VN[Dea]GDAS[Pho]PAAAESGAK)

B

| | Abnormal (≥Mean + 2SD) | Normal | Sensitivity | Specificity |
|---|---|---|---|---|
| Control | 0 | 10 | / | / |
| AD | 4 | 4 | 0.50 | 1.00 |
| FTLD | 4 | 3 | 0.57 | 1.00 |
| ALS | 4 | 6 | 0.40 | 1.00 |

# Fig. 1-2

A

● pSer46-MARCKS
(VN[Dea]GDAS[Pho]PAAAESGAK)

B

| | Abnormal (≥Mean + 2SD) | Normal | Sensitivity | Specificity |
|---|---|---|---|---|
| Healthy control | 0 | 10 | | |
| AD | 3 | 5 | 0.38 | 1.00 |
| FTLD | 3 | 4 | 0.43 | 1.00 |
| ALS | 2 | 8 | 0.2 | 1.00 |

# Fig. 2-1

A

● Non-phosphorylated-MARCKS
(VN[Dea]GDASPAAAESGAK)

B

|  | Abnormal (≥Mean + 2SD) | Normal | Sensitivity | Specificity |
|---|---|---|---|---|
| Control | 1 | 9 | | |
| AD | 0 | 8 | 0.00 | 0.90 |
| FTLD | 3 | 4 | 0.43 | 0.90 |
| ALS | 1 | 9 | 0.10 | 0.90 |

# Fig. 2-2

## A

● Non-phosphorylated-MARCKS
(VN[Dea]GDASPAAAESGAK)

## B

|  | Abnormal (≥Mean + 2SD) | Normal | Sensitivity | Specificity |
|---|---|---|---|---|
| Healthy control | 1 | 9 | | |
| AD | 0 | 8 | 0.00 | 0.90 |
| FTLD | 4 | 3 | 0.57 | 0.90 |
| ALS | 0 | 10 | 0.00 | 0.90 |

## Fig. 3-1

A

pSer46-MARCKS
(VN[Dea]GDAS[Pho]PAAAESGAK)

* (p=0.00358)

* (p=0.0007)

* (p=0.02941)

ppm pSer46-MARCKS
(Relative to total protein)

800

600

400

200

0

Control     AD        FTLD       ALS

n=10       n=8        n=7       n=10

— : median
* : p<0.05 in Wilcoxon rank sum test

B

* (p=0.0128)

% pSer46-MARCKS
(Relative to total MARCKS)

30

20

10

0

Control     AD        FTLD       ALS

n=10       n=8        n=7       n=10

— : median
* : p<0.05 in Wilcoxon rank sum test

# Fig. 3-2

A

□ ● Non-phosphorylated MARCKS
(VN[Dea]GDASPAAAESGAK)

ppm Non-phosphorylated MARCKS (Relative to total protein)

* (p=0.0455)    * (p=0.0212)

400
300
200
100
0

Control    AD    FTLD    ALS
n=10       n=8    n=7     n=10

— : median
* : p<0.05 in Wilcoxon rank sum test

B

% Non-phosphorylated MARCKS (Relative to total MARCKS)

* (p=0.0217)

30
20
10
0

Control    AD    FTLD    ALS
n=10       n=8    n=7     n=10

— : median
* : p<0.05 in Wilcoxon rank sum test

# Fig. 4-1

A  Control

800
700
600
500
400
300
200
100
0

r = 0.6715

y = 0.2822x+21.652

0  100  200  300  400

B  AD

800
700
600
500
400
300
200
100
0

r = -0.385

0  100  200  300  400

C  FTLD

800
700
600
500
400
300
200
100
0

r = 0.2095

0  100  200  300  400

D  ALS

800
700
600
500
400
300
200
100
0

r = 0.1226

0  100  200  300  400

ppm pSer46-MARCKS
(Relative to total protein)

ppm Non-phosphorylated MARCKS
(Relative to total protein)

# Fig. 4-2

# Fig. 4-3

# Fig. 4-4

# Fig. 5-1

r = 0.6739

y = 0.0015x-0.0636

# Fig. 5-2

# Fig. 6-1

pTDP-43, Occipital cortex, Human FTLD patient

20 μm

# Fig. 6-2

A

B

# Fig. 6-3

Human FTLD, Occipital cortex

A: pMARCKS | MAP2 | Merge — 40 μm

B: pMARCKS | pTDP43 | Merge — 40 μm

C: pMARCKS | pTDP43 | Merge+DAPI — 20 μm

# Fig. 7

A  Human FTLD, Frontal cortex  B  Human FTLD, Occipital cortex

C  Human control, Parietal cortex

# Fig. 8-1

$$DO = \sqrt{(pSer46\text{-}MARCKS)^2 + (Non\text{-}phosphorylated\ MARCKS)^2}$$

Objective score = Sensitivity$_{AD}$ + Sensitivity$_{FTLD}$ + Sensitivity$_{ALS}$ + Specificity$_{Common}$

# Fig. 8-2

* p = 0.0128

* p = 0.0022

* p = 0.0367

DO (a.u)

800

600

400

200

0

Control
N=10

AD
N=8

FTLD
N=7

ALS
N=10

Optimal threshold
for diagnosis
(=50.262)

*p<0.05 in Wilcoxon's rank sum test

# Fig. 8-3

Optimal sensitivity and specificity

|  | DO range | Abnormal (DO>50.262) | Normal | Sensitivity | Specificity |
|---|---|---|---|---|---|
| Control | [50.262, 54.239] | 2 | 8 | | |
| AD | | 7 | 1 | 0.875 | 0.800 (0.889) |
| FTLD | | 6 | 1 | 0.857 | 0.800 (0.889) |
| ALS | | 10 | 0 | 1.00 | 0.800 (0.889) |

# Fig. 9

# Fig. 10A

DLB     AD
Occipital lobe    Occipital lobe
∪ Temporal lobe   ∪ Temporal lobe
83    107    87

DLB     AD
Occipital lobe    Occipital lobe
∩ Temporal lobe   ∩ Temporal lobe
17    7    26

DLB     AD
Occipital lobe    Occipital lobe
22    25    86

DLB     AD
Temporal lobe    Occipital lobe
122    45    66

DLB     AD
Occipital lobe    Temporal lobe
31    16    100

DLB     AD
Temporal lobe    Temporal lobe
97    70    46

pSer46-MARCKS is contained    N=5, p<0.05 Benjamini-Hochberg modified welch test

45

# Fig. 10B

| | | Human DLB | | | | Human AD | | | |
|---|---|---|---|---|---|---|---|---|---|
| | | Occipital lobe | | Temporal lobe | | Occipital lobe | | Temporal lobe | |
| Human p-site | Mouse p-site | Mean Ratio | P-value (Welch's test) | Mean Ratio | P-value (Welch's test) | Mean Ratio | P-value (Welch's test) | Mean Ratio | P-value (Welch's test) |
| S27 | S27 | 1.084 | 0.337 | 1.420 | 1.E-04 | 1.062 | 0.511 | 0.985 | 0.758 |
| S46 | S46 | 1.114 | 0.100 | 1.211 | 2.E-07 | 1.321 | 1.E-07 | 0.934 | 0.007 |
| S145 | S138 | 1.093 | 0.801 | 0.544 | 0.288 | 1.637 | 0.093 | 1.313 | 0.290 |
| T150 | T143 | 0.737 | 0.050 | 0.580 | 6.E-04 | 1.481 | 9.E-06 | 1.394 | 2.E-04 |

# Fig. 11A

Occipital cortex of human DLB patient

# Fig. 11B

Occipital cortex of human non-DLB patient

Occipital cortex of human DLB patient

# Fig. 11C

Occipital cortex of human DLB patient

# Fig. 11D

Occipital cortex of human DLB patient

# Fig. 11E

# Fig. 12

pSer46-MARCKS
pSer129-α-Syn

☐ non-Tg sibling (non-Tg)

■ Humanized α-Syn-BAC-Tg/GBA-hetero-KO (Tg)

N=3 (n=30), *p<0.05, **p<0.01
Two-way ANOVA followed by student's t test

# Fig. 13A

# Fig. 13B

| Olfactory bulb　　6 months |
|---|

pSer46-MARCKS — Mitral cell layer / Inner plexiform layer

pSer129-α-Syn — Mitral cell layer / Inner plexiform layer

pSer46-MARCKS pSer129-α-Syn — Mitral cell layer / Inner plexiform layer

10 μm

# Fig. 13C

| Olfactory bulb    24 months |
|---|

pSer46-MARCKS

Mitral cell layer

Inner plexiform layer

pSer129-α-Syn

Mitral cell layer

Inner plexiform layer

pSer46-MARCKS
pSer129-α-Syn

Mitral cell layer

10 μm    Inner plexiform layer

# Fig. 13D

# Fig. 14A

α-Syn BAC Tg/GBA-hetero-KO mouse (24 months old)

Parietal cortex

pSer46-MARCKS
pSer129-α-Syn

50 μm

# Fig. 14B

| α-Syn-BAC-Tg/GBA-hetero-KO mouse, Parietal cortex | | | |
|---|---|---|---|
| non-Tg | α-Syn-BAC-Tg /GBA-hetero KO | non-Tg | α-Syn-BAC-Tg /GBA-hetero KO |

# Fig. 15

# Fig. 16

# Fig. 17A

pSer46-MARCKS
pThr203/Tyr205-ERK1 / pThr183/Tyr185-ERK2 (pERK1/2)

☐ non-Tg sibling (non-Tg)
■ Humanized α-Syn-BAC-Tg/GBA-hetero-KO (Tg)

N=3 (n=30), *p<0.05, **p<0.01
Two-way ANOVA followed by student's t test

# Fig. 17B

pSer46-MARCKS
MAP2

pSer46-MARCKS
GFAP

50 μm

# Fig. 17C

pSer46-MARCKS
pThr202/Tyr204-ERK1 / pThr185/Tyr187-ERK2 (pERK1/2)

□ Non-DLB patient

■ DLB patient

N=5, **p<0.01
Student's t test

# Fig. 17D

# Fig. 17E

Fig. 18

# Fig. 19

α-Syn-BAC-Tg/GBA-hetero-KO mouse (6 months old)

pSer46-MARCKS
pSer129-α-Syn

☐ non-Tg    ■ Humanized α-Syn-BAC-Tg/GBA-hetero-KO (Tg)

N=3 (n=30), *p<0.05, **p<0.01
Student's t test

EP 3 764 102 A1

Fig. 20

α-Syn-BAC-Tg/GBA-hetero-KO mouse (24 months old)

pSer46-MARCKS
pSer129-α-Syn

☐ non-Tg    ■ Humanized α-Syn-BAC-Tg/GBA-hetero-KO (Tg)

N=3 (n=30), *p<0.05, **p<0.01
Student's t test

Frontal cortex

Parietal cortex

Occipital cortex

Hippocampus

Olfactory bulb

Striatum

Hypothalamus

Brainstem

Midbrain (Substantia nigra)

# Fig. 21

## INTERNATIONAL SEARCH REPORT

| International application No. |
|---|
| PCT/JP2019/009370 |

**A. CLASSIFICATION OF SUBJECT MATTER**
Int.Cl. G01N33/68(2006.01)i, G01N27/62(2006.01)i

According to International Patent Classification (IPC) or to both national classification and IPC

**B. FIELDS SEARCHED**

Minimum documentation searched (classification system followed by classification symbols)
Int.Cl. G01N33/68, G01N27/62

Documentation searched other than minimum documentation to the extent that such documents are included in the fields searched
Published examined utility model applications of Japan 1922–1996
Published unexamined utility model applications of Japan 1971–2019
Registered utility model specifications of Japan 1996–2019
Published registered utility model applications of Japan 1994–2019

Electronic data base consulted during the international search (name of data base and, where practicable, search terms used)
CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| A | FUJITA, K. et al., "HMGB1, a pathogenic molecule that induces neurite degeneration via TLR4-MARCKS, is a potential therapeutic target for Alzheimer's disease", SCIENTIFIC REPORTS, 25 August 2016, vol. 6/no. 31895, pp. 1-15, DOI: 10.1038/srep31895 | 1-16 |
| A | WO 2015/099094 A1 (TOKYO MEDICAL AND DENTAL UNIVERSITY) 02 July 2015, paragraphs [0074]-[0082] & US 2017/0182012 A1, paragraphs [0153]-[0165] & EP 3088898 A1 | 1-16 |

☒ Further documents are listed in the continuation of Box C.  ☐ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 28 May 2019 (28.05.2019) | 11 June 2019 (11.06.2019) |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**

International application No.

PCT/JP2019/009370

C (Continuation).    DOCUMENTS CONSIDERED TO BE RELEVANT

| Category* | Citation of document, with indication, where appropriate, of the relevant passages | Relevant to claim No. |
|---|---|---|
| A | TAGAWA, K., "Comprehensive phosphoproteome analysis unravels the core signaling network that initiates the earliest synapsepathology in preclinical Alzheimer's disease brain", HUMAN MOLECULAR GENETICS, 17 September 2014, vol. 24/no. 2, pp. 540-558, DOI:10.1093/hmg/ddu475 | 1-16 |
| A | KIMURA, T. et al., "Phosphorylation of MARCKS in Alzheimer disease brains", Neuroreport, 20 March 2000, vol. 11/no. 4, pp. 869-873 | 1-16 |
| A | US 2013/0196925 A1 (HUENTELMAN, M.) 01 August 2013, paragraphs [0004]-[0006] & WO 2012/006640 A2 | 1-16 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018043641 A
- JP 2018138785 A

**Non-patent literature cited in the description**

- **TAGAWA, K. et al.** *Hum. Mol. Genet.,* 2015, vol. 24, 540-58
- **FUJITA, K. et al.** *Sci. Rep.,* 2016, vol. 6, 31895
- **TAGAWA et al.** *Hum Mol Genet,* 2015, vol. 24, 540-558
- **FUJITA et al.** *Sci Rep,* 2016, vol. 6, 31895
- **FUJITA, K. et al.** *Sci Rep,* 2016, vol. 6, 31895
- **YAMAKADO et al.** *Neurosci Res,* 2012, vol. 73, 173-177
- **SHILOV et al.** *Mol421 Cell Proteomics,* 2007, vol. 6, 1638-1655